# EUROPEAN PATENT APPLICATION

(11) **EP 4 659 729 A1**
(43) Date of publication of application: **10.12.2025**
(21) Application number: 25178688.5
(22) Date of filing: 26.05.2025
(51) Int. Cl.: A61K 6/30, A61K 6/76, A61K 6/889

(54) **DENTAL RESIN-REINFORCED GLASS IONOMER CEMENT COMPOSITION**

(30) Priority: 24.05.2024 JP 2024084587
(71) Applicant: Shofu Inc., Kyoto-shi, Kyoto 605-0983 (JP)
(72) Inventor: TSUKAMOTO, Masahiro, Kyoto, 605-0983 (JP); UCHIDA, Jun, Kyoto, 605-0983 (JP); KIMOTO, Katsuya, Kyoto, 605-0983 (JP)
(74) Representative: Eisenführ Speiser

(57) **Abstract**

To provide a dental resin-reinforced glass ionomer cement composition which causes less stringiness of the mixed material than conventional techniques, is excellent in cavity filling property and in application property to a dental prosthesis device, becomes soon after completion of mixing in a state that it is possible to perform shaping operation, and exhibits excellent mixiability, mechanical characteristic and transparency.

To provide a dental resin-reinforced glass ionomer cement composition comprising
(a) acid-reactive glass powder,
(b) polyalkenoic acid,
(c) water,
(d) polymeraizable monomer,
(e) porous inorganic filler: 1 % by mass or more and 15 % by mass or less, and
(f) polymerization initiator, wherein
the center portion of the (e) porous inorganic filler is an inorganic particle consisting of only silicon dioxide or consisting of silicon dioxide and an oxide containing one or more metal elements.

## Description

### BACKGROUND OF THE INVENTION

### Field of the Invention

The present invention relates to a dental resin-reinforced glass ionomer cement composition for repairing a tooth in which a form was partially lost by mainly caries, breakages and the like or for adhering or luting a dental prosthesis device to a tooth in which a form was partially lost.

### Description of the Related Art

In a dental practice, in order to restore aesthetically and functionally the tooth in which a form was partially lost by caries, breakages and the like, a direct restoration in which a filling material is filled into the tooth and an indirect restoration in which a dental prosthesis device is bonded and/or adhered to a tooth by using a luting material has been performed. One of the representative the filling material and the luting material includes a dental resin-reinforced glass ionomer cement.

Dental resin-reinforced glass ionomer cement contains acid-reactive glass powder represented by fluoroaluminosilicate glass powder, polyalkenoic acid, water, polymerizable monomer and polymerization initiator as the main component, and are mainly in the form of two components, such as a powder-liquid type or a two-paste type. In the dental resin-reinforced glass ionomer cement in each forms of these, a mixed material is prepared immediately before use by performing a hand mixing or a mechanical mixing in the case of the powder-liquid type and by performing a hand mixing or an automatic mixing using a mixing tip in the case of two-paste type, to use. In the dental resin-reinforced glass ionomer cement mixed in this manner, in addition to the acid-base reaction between the polyalkenoic acid and the acid-reactive glass powder, a chemical polymerization reaction of the polymerizable monomer is initiated, and setting progresses. There is also a type that can be set by photopolymerization by further adding a photopolymerization initiator.

Dental resin-reinforced glass ionomer cement have characteristics such as long-term sustained release of fluoride ion, high transparency and mechanical characteristic.

Furthermore, when the light curing ability is imparted, because the composition can be set at the timing intended by the operator by irradiation with light, there is an advantage that there is no need to wait for setting and the like.

Japanese Unexamined Patent Application Publication No. 2021-031409 discloses a technology in which a tri- or higher functional (meth) acrylamide-based polymerizable monomer is further added to the general main component of the above-described dental resin-reinforced glass ionomer cement, thereby achieving high surface setting property, excellent color resistance and small expansion due to water absorption, even when moistened.

### SUMMARY OF THE INVENTION

### Technical Problem

Since a dental resin-reinforced glass ionomer cement contains a high consistency aqueous solution of polyalkenoic acid in its composition, there is a tendency that the mixed material is stringy immediately after mixing. This stringiness can adversely affect the operability of filling and bonding, and the filling operation is particularly affected with this. For example, after filling a mixed material immediately after mixing is filled into a cavity using a dental instrument such as a dental syringe or an instrument, when the dental instrument is pulled away from the mixed material, there is a case where the mixed material becomes stringy to adhere to the surrounding tooth substance or oral mucosa. In such a case, it is necessary to carefully remove the adhesion, which makes the operation complicated.

In addition, since the dental resin-reinforced glass ionomer cement begins to set due to the acid-base reaction and chemical polymerization immediately after mixing, regardless of the properties of the mixed material, after a certain period of time, the stringiness of the mixed material decreases, making it easier to perform shaping operations. Therefore, when shaping is performed on the mixed material filled in the cavity, the shaping operation is performed after waiting until the stringiness of the mixed material is reduced. On the other hand, if the mixed material is extremely stringy and it takes time for the stringiness to decrease, the treatment time is longer and the risk of the treatment site being contaminated by saliva increases.

As the method for reducing stringiness of the mixed material immediately after mixing, there is a method for increasing the ratio or the particle diameter of acid-reactive glass powder contained in the dental resin-reinforced glass ionomer cement. However, when the ratio of the acid-reactive glass powder is increased, the viscosity of the mixed material increases, and thus there is a case where the mixiability decreases. Furthermore, when the particle diameter of the acid-reactive glass powder is increased, the acid-base reactivity between the acid-reactive glass powder and the polyalkenoic acid decreases, and thus there is a case where a decrease in the mechanical characteristic of the set product is caused.

Therefore, an object of the present invention is to provide a dental resin-reinforced glass ionomer cement composition which causes less stringiness of the mixed material than conventional techniques, is excellent in cavity filling property and in application property to a dental prosthesis device, becomes soon after completion of mixing in a state that it is possible to perform shaping operation, and exhibits excellent mixiability, mechanical characteristic and transparency.

### Solution to Problem

The present inventors made an intensive study under the above problems. As a result, the present inventors have found that, by compounding a specific porous inorganic filler in a specific range, the dental resin-reinforced glass ionomer cement composition exhibits little stringiness and therefore is excellent in cavity filling property and is excellent in application property to a dental prosthesis device, and becomes soon after completion of mixing in a state that it is possible to perform shaping operation, and also exhibits excellent mixability, mechanical characteristic and transparency. Furthermore, the present inventors have found that, a dental resin-reinforced glass ionomer cement composition containing an organic-inorganic composite filler in addition to the specific porous inorganic filler within a specific range with respect to the total content and a content ratio has a low viscosity of the mixed material and improves mixability, and, the present invention has been completed.

That is, the above problem can be solved by the following present invention.

A dental resin-reinforced glass ionomer cement composition comprising
(a) acid-reactive glass powder,
(b) polyalkenoic acid,
(c) water,
(d) polymeraizable monomer,
(e) porous inorganic filler: 1 % by mass or more and 15 % by mass or less, and
(f) polymerization initiator, wherein
the center portion of the (e) porous inorganic filler is an inorganic particle consisting of only silicon dioxide or consisting of silicon dioxide and an oxide containing one or more metal elements.

### Advantageous Effects of Invention

The present invention provide a dental resin-reinforced glass ionomer cement composition that exhibits little stringiness immediately after mixing and therefore is excellent in cavity filling property and is excellent in application property to a dental prosthesis device, and becomes soon after completion of mixing in a state that it is possible to perform shaping operation and therefore shorten treatment time, and also exhibits excellent mixability, mechanical characteristic and transparency.

### DETAILED DESCRIPTION OF THE PREFERRED EMBODIMENTS

In the present invention, the (d) porous inorganic filler may have a 50 % particle diameter (D50) within a range of 0.1 µm or more and 10 µm or less, a pore volume within a range of 0.01 cc/g or more and 1.00 cc/g or less, and a specific surface area within a range of 5 m²/g or more and 500 m²/g or less.

In the present invention, the dental resin-reinforced glass ionomer cement composition may further comprise (g) organic-inorganic composite filler,
the (g) organic-inorganic composite filler may have a 50 % particle diameter (D50) within a range of 1 µm or more, and
the (g) organic-inorganic composite filler may contain 10 mass % or more and 85 mass % or less of an inorganic filler having a 50% particle diameter (D50) of 0.01 µm or more and 3 µm or less.

In the present invention, a total content of the (e) porous inorganic filler and the (g) organic-inorganic composite filler may be 2 % by mass or more and 20 % by mass or less based on the whole of the dental resin-reinforced glass ionomer cement composition, and
the mass ratio of the content of the (e) porous inorganic filler to the content of the (g) organic-inorganic composite filler [(g) organic-inorganic composite filler/(e) porous inorganic filler] may be 0.5 or more and 2.5 or less.

In the present invention, the dental resin-reinforced glass ionomer cement composition may comprise
(a) an acid-reactive glass powder: 30 % by mass or more and 80 % by mass or less,
(b) a polyalkenoic acid: 0.5 % by mass or more and 17 % by mass or less,
(c) water: 0.5 % by mass or more and 27 % by mass or less, and
(d) polymeraizable monomer: 2 % by mass or more and 48 % by mass or less.

In the present invention, shaping start time of the dental resin-reinforced glass ionomer cement composition may be within 40 seconds.

The present invention will be described in detail below.

In the present specification, the term "dental resin-reinforced glass ionomer cement composition" means a dental material that sets by a polymerization reaction of a compound having a polymerizable group such as a polymerizable monomer, an oligomer having a polymerizable group and/or a polymer having a polymerizable group, and by an acid-base reaction that occurs between an acid-reactive glass powder and a polyalkenoic acid in the presence of water.

In the present specification, the degree of stringiness of the mixed material is evaluated by the following method. That is, a mixed material of the dental resin-reinforced glass ionomer cement composition (total amount specified as 360 mg) was filled into a simulated cavity of a certain size, the excess was scraped off to make the surface flat, and after 10 seconds from the end of mixing, the cylindrical tip of a metal instrument (diameter: φ1.5 mm) was vertically submerged 0.5 mm into the mixed material, and the instrument was immediately gently pulled up to observe the degree of stringiness. The test is performed under an environment of a temperature of 23±1 °C and a humidity of 50±10%. At this time, when the mixed material does not become stringy or when there is only slight stringiness, it is evaluated as "good mixed material property with little stringiness".

In addition, the degree of stringiness of the mixed material is evaluated using the same method, and the time from the end of mixing to until the stringiness of the mixed material reduced and it becomes possible to perform shaping operation is defined as "shaping start time".

In the present specification, the "contrast ratio" is a measure of transparency expressed as a numerical value between 0 and 1 and indicates that the closer to 0, the higher the transparency, and the closer to 1, the lower the transparency. The contrast ratio is calculated by measuring the Y value (Yw) on a white plate and the Y value (Yb) on a black plate using a colorimeter for 1 mm thick set product, and determining the ratio (Yb/Yw) of these values.

In the present specification, "polyalkenoic acid" means a polymer containing an ethylenically unsaturated monomer unit having an acidic group. In the present specification, the term "(meth)acrylate" inclusively refers to both acrylate and methacrylate, the term "(meth)acryloyl" inclusively refers to both acryloyl and methacryloyl, the term "(meth)acrylic acid" inclusively refers to both acrylic acid and methacrylic acid, and the term "(meth)acrylamide" inclusively refers to both acrylamide and methacrylamide.

In addition, in the present specification, the term "50% particle diameter (D50)" means a particle diameter at which an integrated value from the small particle diameter side becomes 50% in a volume-based particle diameter distribution measured using a laser diffraction/scattering type particle size distribution measuring device and the like.

In the present specification, the term "pore volume" refers to a value determined by the BJH method from an adsorption isotherm obtained by a nitrogen adsorption method.

In the present specification, the term "specific surface area" refers to a value determined by the BET method from an adsorption isotherm obtained by a nitrogen adsorption method.

When using the dental resin-reinforced glass ionomer cement composition of the present invention, for example, a mixing device for capsule for dental restorative material and a capsule for mixing and dispensing dental material can be used. In the dental field, capsules for dental cement have been widely used as container for two-component mixed and mixed dental cements. When using a capsule for dental cement, the two components are mixed and mixed using an automatic mixer such as a capsule mixer, and then a filling tool such as an applier is attached to the capsule, and the mixed material inside the capsule can be filled into the treatment site such as a cavity.

The dental resin-reinforced glass ionomer cement composition of the present invention comprises, as essential components, (a) acid-reactive glass powder, (b) polyalkenoic acid, (c) water, (d) polymerizable monomer, a specific (e) porous inorganic filler, and (f) polymerization initiator, and comprises the (e) porous inorganic filler in a specific amount. By this component configuration, the mixed material exhibits little stringiness and therefore is excellent in cavity filling property and is excellent in application property to a dental prosthesis device, becomes soon after completion of mixing in a state that it is possible to perform shaping operation, and also exhibits excellent mixability mechanical characteristic and transparency.

Furthermore, in the dental resin-reinforced glass ionomer cement composition of the present invention, by further comprising (g) organic-inorganic composite filler, and by setting a total content and a content ratio of (e) porous inorganic filler and (g) organic-inorganic composite filler within a specific range, it is possible that the viscosity of the mixed material is kept low and mixing property is further improved. Hereinafter, the above component of the present disclosure will be described in detail.

### <(a) Acid-reactive glass powder>

The (a) acid-reactive glass powder that can be used in the dental resin-reinforced glass ionomer cement composition of the present invention is a component contributing to the setting of the composition and needs to contain an acid-reactive element such as metal element, and fluorine element. Because the (a) acid-reactive glass powder contains an acid reactive element, the acid-base reaction of the (a) acid-reactive glass powder with the acid group contained in the (b) polyalkenic acid progresses in the presence of (c) water. Specific examples of the acid reactive element include sodium, potassium, calcium, strontium, barium, lanthanum, aluminum and zinc, but are not limited thereto. One or two or more kinds of these acid reactive elements may be contained and a content thereof is not particularly limited.

Further, it is preferable that the (a) acid-reactive glass powder includes an X-ray impermeable element in order to impart X-ray contrast property to the dental resin-reinforced glass ionomer cement composition of the present invention. Specific examples of the X-ray impermeable element include strontium, lanthanum, zirconium, titanium, yttrium, ytterbium, tantalum, tin, tellurium, tungsten and bismuth, but are not limited thereto. In addition, other element contained in the (a) acid-reactive glass powder is not particularly limited and the (a) acid-reactive glass powder in the present invention may include various elements.

Examples of the (a) acid-reactive glass powder include aluminosilicate glass, borosilicate glass, aluminoborate glass, boro aluminosilicate glass, phosphate glass, borate glass, silicate glass which contain the above described acid reactive element, fluorine element and X-ray impermeable element, but are not limited thereto.

Further, a particle shape of the (a) acid-reactive glass powder is not particularly limited, but arbitral particle shapes such as spherical, needle-like, plate-like, ground-like, and scaly-shape may be used without any limitation. These (a) acid-reactive glass powder may be used alone or in a combination of a few kinds thereof.

A manufacturing method of the (a) acid-reactive glass powder is not particularly limited, and any of the (a) acid-reactive glass powder manufactured by any manufacturing methods such as a melting method, a vapor phase method and a sol-gel method may be used without any problem. Among them, the (a) acid-reactive glass powder manufactured by a melting method or a sol-gel method which can easily control a kind of element and the content thereof is preferably used.

The (a) acid-reactive glass powder may be ground to use in order to obtain a desirable particle diameter. A grinding method is not particularly limited, but an acid-reactive glass powder obtained by grinding which use any of wet or dry grinding methods may be used. Specifically, the particle diameter may be appropriately adjusted according to the desired property to be imparted to the dental resin-reinforced glass ionomer cement composition of the present invention by grounding a raw material glass with a high speed rotating mill such as a hammer mill and a turbo-mill, a container driving type mill such as a ball mill, a planetary mill and a vibration mill, a medium stirring mill such as an attritor and a bead mill and a jet mill and the like.

It is preferable that the 50% particle diameter (D50) of the (a) acid-reactive glass powder is 0.5 µm or more and 20 µm or less, and more preferably 2.0 µm or more and 15 µm or less. The dental resin-reinforced glass ionomer cement composition of the present invention may contain only acid-reactive glass powder having the 50% particle diameter (D50) of 0.5 µm or more and 20 µm or less as the (a) acid-reactive glass powder.

When the 50% particle diameter (D50) of the (a) acid-reactive glass powder is less than 0.5 µm, the surface area thereof increases and it becomes impossible to contain the acid-reactive glass powder in a large amount into the composition, therefore there is a case where the mechanical characteristic decreases. Further there is a case where working time is remarkably shortened. When the 50% particle diameter (D50) of the (a) acid-reactive glass powder exceeds 20 µm, there is a case where the mechanical characteristic decreases. In the case of using as a material for filling, the surface of the material after polishing becomes rough, therefore there is a risk of staining in the oral cavity. Further, in case of using as a material for luting, because the film thickness becomes thick, the attached or adhered dental prosthesis device is lifted and therefore there is a case where the intended fit cannot be obtained.

The (a) acid-reactive glass powder may be treated with various surface treatments, heat treatment, aggregating treatment in a liquid phase or a vapor phase and the like to such a range that the acid-base reaction of the (a) acid-reactive glass powder with the (b) polyalkenoic acid is not adversely affected, in order to adjust operability, a setting characteristic, a mechanical characteristic and the like of the dental resin-reinforced glass ionomer cement composition of the present invention. These treatments can be performed alone or in a combination of a few kinds thereof, and the order in which the treatments are performed is not particularly limited. Among them, the surface treatment and the heat treatment are preferable because it is easy to control various characteristics and those are superior in productivity.

Specific examples of the surface treatment of the (a) acid-reactive glass powder include washing with an acid such as phosphoric acid or acetic acid, surface treatment with an acidic compound such as tartaric acid or polycarboxylic acid, surface treatment with a fluoride such as aluminum fluoride and surface treatment with a silane compound such as (meth) acryloyloxymethyl trimethoxysilane, 3-(meth) acryloyloxypropyl trimethoxysilane, 8-(meth) acryloyloxyoctyl trimethoxysilane, 3-mercaptopropyl trimethoxysilane, tetramethoxysilane, tetraethoxysilane, partially hydrolyzed oligomer of tetramethoxysilane and partially hydrolyzed oligomer of tetraethoxysilane. The surface treatment is not limited the above described surface treatment and these surface treatments can be used alone or in a combination thereof. Furthermore, the amount of the surface treatment agent relative to the (a) acid-reactive glass powder when performing the surface treatment is not particularly limited, and may be appropriately adjusted depending on the particle diameter of the (a) acid-reactive glass powder and desired property.

Specific examples of the heat treatment of the (a) acid-reactive glass powder include a treatment method which includes heating for a range of 1 hour to 72 hours within a range of 200 °C or more to 800 °C or less using an electric furnace. The heat treatment which can be used in the present invention is not limited the above described treatment and the treatment process may be a processing at uni-temperature or a multi-stage processing at multi-temperatures.

It is preferable that a content of the (a) acid-reactive glass powder is 30 % by mass or more to 80 % by mass or less mass% and more preferably 45 % by mass or more and 80 % by mass or less based on the whole of the dental resin-reinforced glass ionomer cement composition of the present invention. When the content of the (a) acid-reactive glass powder is less than 30 % by mass, there is a case where the mechanical characteristic decrease. Furthermore, when the content of the (a) acid-reactive glass powder exceeds % by mass, there is a case where operability is adversely affected such as a case where working time becomes remarkably shorter or a case where the viscosity of the mixed material increases, resulting in poor mixing property.

### <(b) Polyalkenoic Acid>

The (b) polyalkenoic acid that can be used in the dental resin-reinforced glass ionomer cement composition of the present invention is a component contributing to the set of the composition. Any polyalkenoic acids can be used without any limitation as the (b) polyalkenoic acid, as long as it is a homopolymer or copolymer of an ethylenically unsaturated monomer having at least one or more carboxy groups in the molecule such as an ethylenically unsaturated monocarboxylic acid, an ethylenically unsaturated dicarboxylic acid, an ethylenically unsaturated tricarboxylic acid and the like. Further, the (b) polyalkenoic acid may be a copolymer of an ethylenically polymerizable monomer having no carboxy group and/or an ethylenically unsaturated monomer having an acidic group other than a carboxy group, such as a phosphoric acid group, a phosphonic acid group, or a sulfonic acid group, and an ethylenically unsaturated monomer having a carboxy group, in the molecule, without any problems. In such a copolymer, the content of ethylenically unsaturated monomer units having a carboxy group is preferably 60 % or more, more preferably 70 % or more, and most preferably 80 % or more.

Specific examples of the ethylenically unsaturated monomer having a carboxy group that can be used to obtain the (b) polyalkenoic acid include ethylenically unsaturated monocarboxylic acid such as acrylic acid, methacrylic acid, 2-chloroacrylic acid, 3-chloroacrylic acid and 2-cyanoacrylic acid; ethylenically unsaturated dicarboxylic acids such as mesaconic acid, maleic acid, maleic anhydride, itaconic acid, itaconic anhydride, fumaric acid, glutaconic acid and citraconic acid; ethylenically unsaturated tricarboxylic acids such as aconitic acid, 1-butene-1,2,4-tricarboxylic acid and 3-butene-1,2,3-tricarboxylic acid, but are not limited thereto. Among them, it is preferable to use the (b) polyalkenoic acid synthesized from only acrylic acid as a starting material or the (b) polyalkenoic acid synthesized from two or more kinds of starting materials such as acrylic acid and maleic acid, acrylic acid and maleic anhydride, acrylic acid and itaconic acid, and acrylic acid and 3-butene-1,2,3-tricarboxylic acid.

The polymerization method used for obtaining various (b) polyalkenic acid is not particularly limited, and a polymer polymerized by any methods such as solution polymerization, suspension polymerization, emulsion polymerization or the like, may be used without any limitation. In addition, as a polymerization initiator and a chain transfer agent which is used at polymerization, a known polymerization initiator and a known chain transfer agent can be used, and the additive amounts thereof may be appropriately adjusted depending on the desired characteristics. The (b) polyalkenic acid obtained by such way can be used alone, or in a combination of a few kinds thereof.

A weight average molecular weight of the (b) polyalkenoic acid is preferably within a range of 30,000 or more and 300,000 or less. Herein, the weight average molecular weight means the average molecular weight which is calculated based on molecular weight distribution measured by gel permeation chromatography. When the weight average molecular weight of the (b) polyalkenoic acid is less than 30,000, there is a case where the mechanical characteristic decreases. In addition, when the weight average molecular weight of the (b) polyalkenoic acid is more than 300,000, there is a case where operability is adversely affected such as a case where the working time becomes remarkably shorter, or there is a case where the viscosity of the mixed material increases, resulting in poor mixing property. The dental resin-reinforced glass ionomer cement composition of the present invention may contain only polyalkenoic acid with a weight average molecular weight within a range of 30,000 to 300,000 as the (b) polyalkenoic acid.

In addition, the (b) polyalkenoic acid may be used after some of its carboxy groups have been neutralized with a basic compound for the purpose of adjusting the acid-base reactivity with the (a) acid-reactive glass powder as long as a range that various properties are not adversely affected. Examples of the basic compound used for neutralization include alkali metal hydroxides such as sodium hydroxide, potassium hydroxide and lithium hydroxide; alkali metal carbonates such as sodium carbonate, potassium carbonate and lithium carbonate; and alkali metal hydrogen carbonates such as sodium hydrogen carbonate, potassium hydrogen carbonate and lithium hydrogen carbonate. Furthermore, various amine compounds such as primary amines, secondary amines and tertiary amines may be used without any problem. As the amine compound, triethanolamine, diethanolamine, N-methyldiethanolamine, 2-dimethylaminoethyl (meth) acrylate and the like can be suitably used.

It is preferable that a content of the (b) polyalkenoic acid is 0.5 % by mass or more and 17 % by mass or less and more preferably 3 % by mass or more and 11 % by mass or less based on the whole of the dental resin-reinforced glass ionomer cement composition of the present invention. When the content of the (b) polyalkenoic acid is less than 0.5 % by mass, there is a case where the mechanical characteristic decrease. When the content of the (b) polyalkenoic acid is more than 17 % by mass, there is a case where operability is adversely affected such as a case where the working time becomes remarkably shorter or a case where the viscosity of the mixed material increases, resulting in poor mixing property.

### <(c) Water>

The (c) water that can be used in the dental resin-reinforced glass ionomer cement composition of the present invention is a component which functions as a solvent for dissolving the (b) polyalkenoic acid, diffuses metal ions eluted from the (a) acid-reactive glass powder, and induces a cross-linking reaction with the (b) polyalkenoic acid.

Any water can be used as the (c) water as long as it does not contain impurities inhibiting the acid-base reaction and adversely affecting on the settability and the mechanical characteristic of the dental resin-reinforced glass ionomer cement composition of the present invention without any limitations. Specifically, it is preferably to use distilled water or ion-exchanged water.

It is preferable that a content of the (c) water is 0.5 % by mass or more and 27 % by mass or less and more preferably 2 % by mass or more and 14 % by mass or less based on the whole of the dental resin-reinforced glass ionomer cement composition of the present invention. When the content of the (b) water is less than 0.5 % by mass, there is a case where operability is adversely affected such as a case where working time becomes remarkably shorter or a case where the viscosity of the mixed material increases, resulting in poor mixing property. When the content of the (c) water exceeds 27 % by mass, there is a case where the mechanical characteristic decrease.

### <(d) Polymerizable monomer>

The (d) polymerizable monomer that can be used in the dental resin-reinforced glass ionomer cement composition of the present invention is a component contributing to the setting of the composition. As the (d) polymerizable monomer, any known polymerizable monomer can be used without particularly limitation in terms of its molecular structure. That is, specific examples of a polymerizable unsaturated group contained in the (d) polymerizable monomer include a (meth) acryloyloxy group, a (meth) acrylamide group, a styryl group, a vinyl group and an allyl group, but are not limited thereto. Among these polymerizable unsaturated groups, a (meth) acryloyloxy group and a (meth) acrylamide group are preferable because of its excellent polymerization rate. In addition, there is no particular limitation on the number of polymerizable unsaturated groups contained in the (d) polymerizable monomer. The hydrocarbon group bonded to the polymerizable unsaturated group may be any of an aliphatic hydrocarbon group, an alicyclic hydrocarbon group, an aromatic hydrocarbon group and a combination thereof, and the hydrocarbon group may have any substituent such as an acidic group, a hydroxyl group, a halogen atom, an alkoxy group, an amino group and a glycidyl group. Specific examples of the (d) polymerizable monomer are as follows.

Specific examples of the monofunctional polymerizable monomer include (meth) acrylic acid esters such as (meth) acrylic acid, methyl (meth) acrylate, ethyl (meth) acrylate, isopropyl (meth) acrylate, n-propyl (meth) acrylate, isobutyl (meth) acrylate, n-butyl (meth) acrylate, t-butyl (meth) acrylate, sec-butyl (meth) acrylate, n-amyl (meth) acrylate, isoamyl (meth) acrylate, n-hexyl (meth) acrylate, isodecyl (meth) acrylate, lauryl (meth) acrylate, stearyl (meth) acrylate, 2-ethylhexyl (meth) acrylate, cyclohexyl (meth) acrylate, adamantyl (meth) acrylate, phenyl (meth) acrylate, phenoxy diethyleneglycol (meth) acrylate, methoxy polyethylene glycol (meth) acrylate, benzyl (meth) acrylate, 2-phenylethyl (meth) acrylate, o-phenoxybenzyl (meth) acrylate, m-phenoxybenzyl (meth) acrylate, p-phenoxybenzyl (meth) acrylate, tetrahydrofurfuryl (meth) acrylate, glycidyl (meth) acrylate, isobornyl (meth) acrylate, allyl (meth) acrylate, 2-methoxyethyl (meth) acrylate, 2-ethoxyethyl (meth) acrylate, phenoxyethyl (meth) acrylate, 2-hydroxyethyl (meth) acrylate, 2-hydroxypropyl (meth) acrylate, 3-hydroxypropyl (meth) acrylate, 2-hydroxybutyl (meth) acrylate, glycerol (meth) acrylate, (meth) acryloyloxyethyl methyl succinate, 2-(meth) acryloyloxyethyl propionate, acetoacetoxyethyl (meth) acrylate, acetoacetoxypropyl (meth) acrylate and acetoacetoxybutyl (meth) acrylate; silane compounds such as 3-(meth) acryloyloxypropyl trimethoxysilane and 3-(meth) acryloyloxypropyl triethoxysilane; amines such as 2-(N,N-dimethylamino) ethyl (meth) acrylate and 2-(N,N-diethylamino) ethyl (meth) acrylate; fluorine-containing (meth) acrylates such as 2,2,2-trifluoroethyl (meth) acrylate, perfluorohexylethyl (meth) acrylate and perfluorooctylethyl (meth) acrylate and (meth) acrylamides thereof; and N-methylol (meth) acrylamide.

Specific examples of the aromatic bifunctional polymerizable monomer include 2,2-bis [4-[3-(meth) acryloyloxy-2-hydroxypropoxy] phenyl]propane, 2,2-bis (4-(meth) acryloyloxyphenyl) propane, 2,2-bis (4-(meth) acryloyloxy ethoxyphenyl) propane, 2,2-bis (4-(meth) acryloyloxy diethoxyphenyl) propane, 2,2-bis (4-(meth) acryloyloxy tetraethoxyphenyl) propane, 2,2-bis (4-(meth) acryloyloxy pentaethoxyphenyl) propane, 2,2-bis (4-(meth) acryloyloxy dipropoxyphenyl) propane, 2-(4-(meth) acryloyloxy ethoxyphenyl)-2-(4-(meth) acryloyloxy diethoxyphenyl) propane, 2-(4-(meth) acryloyloxy diethoxyphenyl)-2-(4-(meth) acryloyloxy triethoxyphenyl) propane, 2-(4-(meth) acryloyloxy dipropoxyphenyl)-2-(4-(meth) acryloyloxy triethoxyphenyl) propane, 2,2-bis (4-(meth) acryloyloxy dipropoxyphenyl) propane, 2,2-bis (4-(meth) acryloyloxy isopropoxyphenyl) propane, 2,2-bis (4-(meth) acryloyloxy polyethoxyphenyl) propane, 9,9-bis [4-(2-(meth) acryloyloxy ethoxy) phenyl] fluorene, and (meth) acrylamides thereof.

Specific examples of the aliphatic bifunctional polymerizable monomer include ethylene glycol di (meth) acrylate, diethylene glycol di (meth) acrylate, triethylene glycol di (meth) acrylate, tetraethylene glycol di (meth) acrylate, polyethylene glycol di (meth) acrylate, dipropylene glycol di (meth) acrylate, tripropylene glycol di (meth) acrylate, neopentyl glycol di (meth) acrylate, 3-methyl-1,5-pentanediol di (meth) acrylate, 1,3-butanediol di (meth) acrylate, 1,4-butanediol di (meth) acrylate, 1,6-hexanediol di (meth) acrylate, 1,9-nonanediol di (meth) acrylate, 1,10-decanediol di (meth) acrylate, tricyclodecane dimethanol di (meth) acrylate, glycerol-1,3-dimethacrylate, 3-hydroxypropyl-1,2-di (meth) acrylate, 2-hydroxy-3-acryloyloxypropyl (meth) acrylate, 1,2-bis (3-(meth) acryloyloxy-2-hydroxypropoxy) ethane, 1,2-bis (3-(meth) acryloyloxy-2-hydroxypropoxy) propane, 2-hydroxy-1,3-bis (3-(meth) acryloyloxy-2-hydroxypropoxy) propane, and (meth)acrylamides thereof.

Specific examples of the tri or more functional polymerizable monomer include trimethylolpropane tri (meth) acrylate, trimethylolethane tri (meth) acrylate, trimethylolmethane tri (meth) acrylate, pentaerythritol tri (meth) acrylate, glycerin tri (meth) acrylate, pentaerythritol tetra (meth) acrylate, dipentaerythritol tetra (meth) acrylate, dipentaerythritol penta (meth) acrylate, dipentaerythritol hexa (meth) acrylate, ditrimethylolpropane tetra (meth) acrylate and (meth) acrylamides thereof.

Specific examples of the urethane-based polymerizable monomer include (meth) acrylate compounds having a urethane linkage, which are derived from an adduct of a polymerizable monomer having a hydroxyl group such as 2-hydroxyethyl (meth) acrylate, 2-hydroxypropyl (meth) acrylate and 3-chloro-2-hydroxypropyl (meth) acrylate, and an isocyanate compound such as methylcyclohexane diisocyanate, methylene bis (4-cyclohexyl isocyanate), hexamethylene diisocyanate, trimethylhexamethylene diisocyanate, isophorone diisocyanate, diisocyanate methylmethylbenzene and 4,4-diphenylmethane diisocyanate.

Because of excellent compatibility with other hydrophilic component, as the (d) polymerizable monomer, it is more preferable to use a hydroxyl group-containing polymerizable monomer having at least one hydroxyl group and at least one of (meth) acryloyloxy group or (meth) acrylamide group as a polymerizable unsaturated group in the molecule. Specific examples of the hydroxyl group-containing polymerizable monomer are as follows.

Specific examples of the hydroxyl group-containing polymerizable monomer include monofunctional polymerizable monomers such as 2-hydroxyethyl (meth) acrylate, 2-hydroxypropyl (meth) acrylate, 3-hydroxypropyl (meth) acrylate, 2-hydroxybutyl (meth) acrylate, 5-hydroxypentyl (meth) acrylate, 6-hydroxyhexyl (meth) acrylate, 10-hydroxydecyl (meth) acrylate, diethylene glycol mono (meth) acrylate, triethylene glycol mono (meth) acrylate, tetraethylene glycol mono (meth) acrylate, polyethylene glycol mono (meth) acrylate, dipropylene glycol mono (meth) acrylate, polypropylene glycol mono (meth) acrylate, 2,3-dihydroxypropyl (meth) acrylate, glycerin mono (meth) acrylate, erythritol mono (meth) acrylate and addition products of phenols and glycidyl (meth) acrylate such as 2-hydroxy-3-phenoxypropyl (meth) acrylate and 2-hydroxy-3-naphthoxypropyl (meth) acrylate, and (meth) acrylamides thereof; and polyfunctional polymerizable monomers such as glycerol-1,3-dimethacrylate, 3-hydroxypropyl-1,2-di (meth) acrylate, bisphenol A diglycidyl (meth) acrylate and 2-hydroxy-3-acryloyloxypropyl (meth) acrylate, and (meth) acrylamides thereof. Further, specific examples include polyfunctional polymerizable monomers in which two or more hydroxyl groups of sugar alcohols (erythritol, arabinitol, xylitol, ribitol, iditol, galactitol, sorbitol, mannitol and the like), monosaccharides (arabinose, xylose, mannose, galactose, fructose and the like), disaccharides (sucrose, maltose, lactose, trehalose and the like) and trisaccharides (maltotriose, raffinose and the like) are substituted with (meth) acryloyloxy groups or (meth) acrylamide groups.

In addition, as the (d) polymerizable monomer, if desired, an acidic group-containing polymerizable monomer containing at least one acidic group in the molecule can be used in order to improve adhesive property to tooth substance, base metal, alumina, zirconia and the like. Specific examples of the acidic group of the acidic group-containing polymerizable monomer include a phosphate group, a pyrophosphate group, a phosphonate group, a carboxyl group, a sulphonate group, and a thiophosphate group. Specific examples of the acidic group-containing polymerizable monomer are as follows.

Specific examples of a phosphate group-containing polymerizable monomer include (meth) acryloyloxymethyl dihydrogen phosphate, 2-(meth) acryloyloxyethyl dihydrogen phosphate, 3-(meth) acryloyloxypropyl dihydrogen phosphate, 4-(meth) acryloyloxybutyl dihydrogen phosphate, 5-(meth) acryloyloxypentyl dihydrogen phosphate, 6-(meth) acryloyloxyhexyl dihydrogen phosphate, 7-(meth) acryloyloxyheptyl dihydrogen phosphate, 8-(meth) acryloyloxyoctyl dihydrogen phosphate, 9-(meth) acryloyloxynonyl dihydrogen phosphate, 10-(meth) acryloyloxydecyl dihydrogen phosphate, 11-(meth) acryloyloxyundecyl dihydrogen phosphate, 12-(meth) acryloyloxydodecyl dihydrogen phosphate, 16-(meth) acryloyloxyhexadecyl dihydrogen phosphate, 20-(meth) acryloyloxyeicosyl dihydrogen phosphate, bis [2-(meth) acryloyloxyethyl] hydrogensphosphate, bis [3-(meth) acryloyloxypropyl] hydrogen phosphate, bis [4-(meth) acryloyloxybutyl] hydrogen phosphate, bis [6-(meta) acryloyloxyhexyl] hydrogen phosphate, bis [8-(meth) acryloyloxyoctyl] hydrogen phosphate, bis [9-(meth) acryloyloxynonyl] hydrogen phosphate, bis [10-(meth) acryloyloxydecyl] hydrogen phosphate, 1,3-di (meth) acryloyloxypropyl-2-dihydrogenphosphate, 2-(meth) acryloyloxy ethylphenyl hydrogen phosphate, 2-(meth) acryloyloxyethyl-2'-bromoethyl hydrogen phosphate, 2-(meth) acryloyloxyethyl-(4-methoxyphenyl) hydrogen phosphate and 2-(meth) acryloyloxypropyl-(4-methoxyphenyl) hydrogen phosphate.

Specific examples of a pyrophosphate group-containing polymerizable monomer include bis [2-(meth) acryloyloxyethyl] pyrophosphate, bis [3-(meth) acryloyloxypropyl] pyrophosphate, bis [4-(meth) acryloyloxybutyl] pyrophosphate, bis [5-(meth) acryloyloxypentyl] pyrophosphate, bis [6-(meth) acryloyloxyhexyl] pyrophosphate, bis [7-(meth) acryloyloxyheptyl] pyrophosphate, bis [8-(meth) acryloyloxyoctyl] pyrophosphate, bis [9-(meth) acryloyloxynonyl] pyrophosphate, bis [10-(meth) acryloyloxydecyl] pyrophosphate, bis [12-(meth) acryloyloxydodecyl] pyrophosphate, tris [2-(meth) acryloyloxyethyl] pyrophosphate and tetra [2-(meth) acryloyloxyethyl] pyrophosphate.

Specific examples of a phosphonate group-containing polymerizable monomer include, 5-(meth) acryloyloxy pentyl-3-phosphonopropionate, 6-(meth) acryloyloxy hexyl-3-phosphonopropionate, 10-(meth) acryloyloxy decyl-3-phosphonopropionate, 6-(meth) acryloyloxy hexyl-3-phosphonoacetate, 10-(meth) acryloyloxy decyl-3-phosphonoacetate and (meth) acryloyloxyethyl phenylphosphonate.

Specific examples of a carboxyl group-containing polymerizable monomer include (meth) acrylic acid, 2-chloro acrylic acid, 3-chloro (meth) acrylic acid, 2-cyano acrylic acid, 1,4-di (meth) acryloyloxyethyl pyromellitic acid, 6-(meth) acryloyloxy naphthalene-1,2,6-tricarboxylic acid, 1-buten-1,2,4-tricarboxylic acid, 3-buten-1,2,3-tricarboxylic acid, N-(meth) acryloyl-p-aminobenzoic acid, N-(meth) acryloyl-5-aminosalicylic acid, 4-(meth) acryloyloxyethyl trimellitic acid and anhydride thereof, 4-(meth) acryloyloxybutyl trimellitic acid and anhydride thereof, 2-(meth) acryloyloxybenzoic acid, β-(meth) acryloyloxyethyl hydrogen succinate, β-(meth) acryloyloxyethyl hydrogen maleate, 11-(meth) acryloyloxy-1,1-undecane dicarboxylic acid, p-vinylbenzoic acid, 4-(meth) acryloyloxy ethoxycarbonyl phthalic acid, 4-(meth) acryloyloxy butyloxycarbonyl phthalic acid, 4-(meth) acryloyloxy hexyloxycarbonyl phthalic acid, 4-(meth) acryloyloxy octyloxycarbonyl phthalic acid, 4-(meth) acryloyloxy decyloxycarbonyl phthalic acid and anhydride thereof, 5-(meth) acryloylamino pentylcarboxylic acid, 6-(meth) acryloyloxy-1,1-hexanedicarboxylic acid, 8-(meth) acryloyloxy-1,1-octanedicarboxylic acid, 10-(meth) acryloyloxy-1,1-decanedicarboxylic acid, and 11-(meth) acryloyloxy-1,1-undecanedicarboxylic acid.

Specific examples of a sulphonate group-containing polymerizable monomer include 2-(meth) acrylamido-2-methylpropanesulfonic acid, styrenesulfonic acid, 2-sulfoethyl (meth) acrylate, 4-(meth) acryloyloxy benzenesulfonic acid and 3-(meth) acryloyloxy propanesulfonic acid.

Specific examples of a thiophosphate group-containing polymerizable monomer include 2-(meth) acryloyloxyethyl dihydrogen thiophosphate, 3-(meth) acryloyloxypropyl dihydrogen thiophosphate, 4-(meth) acryloyloxybutyl dihydrogen thiophosphate, 5-(meth) acryloyloxypentyl dihydrogen thiophosphate, 6-(meth) acryloyloxyhexyl dihydrogen thiophosphate, 7-(meth) acryloyloxyheptyl dihydrogen thiophosphate, 8-(meth) acryloyloxyoctyl dihydrogen thiophosphate, 9-(meth) acryloyloxynonyl dihydrogen thiophosphate, 10-(meth) acryloyloxydecyl dihydrogen thiophosphate, 11-(meth) acryloyloxyundecyl dihydrogen thiophosphate, 12-(meth) acryloyloxydodecyl dihydrogen thiophosphate, 16-(meth) acryloyloxyhexadecyl dihydrogen thiophosphate and 20-(meth) acryloyloxyeicosyl dihydrogen thiophosphate.

Among these acidic group-containing polymerizable monomers, it is preferable to use a carboxyl group-containing polymerizable monomer because of its excellent effect of improving adhesive property, and it is more preferable to use a polymerizable monomer having two or more carboxyl groups. The dental resin-reinforced glass ionomer cement composition of the present invention may not contain an acidic group-containing polymerizable monomer as the (d) polymerizable monomer.

Furthermore, as the (d) polymerizable monomer, a polymerizable monomer having a sulfur atom in the molecule, a polymerizable monomer having a fluoro group, and an oligomer or a polymer having at least one polymerizable group can be used. The (d) polymerizable monomer is not limited the above described and may be used alone or in a combination of plurality thereof.

It is preferable that a content of the (d) polymerizable monomer is 2 % by mass or more and 48 % by mass or less and more preferably 4 % by mass or more and 36 % by mass or less based on the whole of the dental resin-reinforced glass ionomer cement composition of the present invention. When the content of the (d) polymerizable monomer is less than 2 % by mass, there is a case where the chemical polymerization reaction and/or the photopolymerization reaction do not proceed sufficiently, and therefore the mechanical characteristic decreases. Furthermore, when the content of the (d) polymerizable monomer exceeds 48 % by mass, there is a case where the compatibility of the (b) polyalkenoic acid, the (c) water and the (d) polymerizable monomer deteriorates and the set product becomes nonuniform, and therefore the mechanical characteristic and the transparency decrease.

### <(e) Porous inorganic filler>

The (e) porous inorganic filler that can be used in the dental resin-reinforced glass ionomer cement composition of the present invention is an inorganic filler having at least one or more pores and a component for reducing stringiness of the mixed material in the composition. The presence or absence of pores in an inorganic filler can be measured, for example, by gas adsorption method or mercury intrusion method. More specifically, in the present specification, the (d) porous inorganic filler refers to a filler having a pore volume of 0.01 cc/g or greater as measured by gas adsorption. The dental resin-reinforced glass ionomer cement composition of the present invention may contain no fillers other than the (e) porous inorganic filler.

A shape of the (e) porous inorganic filler is not particularly limited, but is preferably spherical or ground-like, because of relatively little effect on the viscosity of the mixed product of the dental resin-reinforced glass ionomer cement composition of the present invention. Furthermore, the 50% particle diameter (D50) of the (e) porous inorganic filler is preferably 0.1 µm or more and 10 µm or less, and more preferably 1 µm or more and 8 µm or less. When the 50% particle diameter (D50) of the (d) porous inorganic filler exceeds 10 µm, there is a case where the mechanical property of the dental resin-reinforced glass ionomer cement composition of the present invention is reduced. Furthermore, when the 50% particle diameter (D50) is less than 0.1 µm, there is a case where the mixing property and property of the mixed product are adversely affected.

The pore volume of the (e) porous inorganic filler is preferably 0.01 cc/g or more and 1.00 cc/g or less, and more preferably 0.08 cc/g or more and 0.80 cc/g or less. Furthermore, the specific surface area of the (e) porous inorganic filler is preferably 5 m²/g or more and 500 m²/g or less, and more preferably 10 m²/g or more and 300 m²/g or less. Because the (e) porous inorganic filler has such properties, it is possible to effectively reduce stringiness of the mixed product in the dental resin-reinforced glass ionomer cement composition of the present invention. When the pore volume and/or specific surface area of the (e) porous inorganic filler is less than the above range, there is a case where the effect of reducing stringiness of the mixed product is difficult to exhibit. Furthermore, when the pore volume and/or specific surface area exceeds the above range, there is a case where the mechanical property is reduced. The dental resin-reinforced glass ionomer cement composition of the present invention may contain only a porous inorganic filler having a 50% particle diameter (D50) of 0.1 µm or more and 10 µm or less, a pore volume of 0.01 cc/g or more and 1.00 cc/g or less, and a specific surface area of 5 m²/g or more and 500 m²/g or less, as the (e) porous inorganic filler.

The (e) porous inorganic filler has at the center portion an inorganic particle consisting of only silicon dioxide or consisting of silicon dioxide and an oxide containing one or more metal elements. Examples of oxides containing metal elements include, but are not limited to, oxides of metal elements such as Al, Ba, Bi, Ca, Ce, Co, Cu, Er, Fe, Hf, Ho, In, La, Mg, Mn, Nd, Ni, Pb, Sb, Sn, Sr, Ta, Ti, Y, Yb, Zn, and Zr. Among these, oxides of Al, Ba, Ca, Co, Cu, Fe, Hf, La, Mg, Ni, Sr, Ti, Zn, and Zr are preferable, and oxides of Ba, Ti, Zr and the like are more preferable, and oxide of Zr is furthermore preferable. The dental resin-reinforced glass ionomer cement composition of the present invention may contain, as the oxide containing a metal element constituting the inorganic particle of the center portion of the (e) porous inorganic filler, only oxides of Al, Ba, Ca, Co, Cu, Fe, Hf, La, Mg, Ni, Sr, Ti, Zn, or Zr or may contain only oxides of Ba, Ti, or Zr, or may contain only oxide of Zr.

The content of the oxide of the metal element contained in the inorganic particles at the center portion of the (e) porous inorganic filler is preferably 30 % by mass or less, in terms of oxide. When the content of the oxide of the metal element exceeds 30% by mass, there is a case where the refractive index of the (e) porous inorganic filler becomes too high, which causes the dental resin-reinforced glass ionomer cement composition of the present invention to become opaque.

The (e) inorganic particles at the center portion of the porous inorganic filler may be manufactured, for example, by mixing an acidic silicic acid liquid, a silicon dioxide sol and optionally one or more aqueous metal salt solutions, spray-drying the resulting mixed slurry, and then heat-treating the resulting dried particles. Detailed production methods for the (e) porous inorganic filler are disclosed in, for example, JP 2019-189637 A, but are not limited to these production methods.

Furthermore, in order to adjust the fluidity of the powder material and paste property in the dental resin-reinforced glass ionomer cement composition of the present invention, the surface of the inorganic particle of the center portion of the (e) porous inorganic filler can be optionally treated with a silane compound such as 3-(meth) acryloyloxymethyl trimethoxysilane, 3-(meth) acryloyloxypropyl trimethoxysilane, 8-(meth) acryloyloxyoctyl trimethoxysilane, 3-mercaptopropyl trimethoxysilane, 3-aminopropyl trimethoxysilane, or 3-glycidoxypropyl trimethoxysilane. Surface treatment agent is not limited to the above described agent, and these surface treatment agents can be used alone or in combination thereof. Furthermore, the amount of surface treatment agent relative to the inorganic particle of the center portion of the (e) porous inorganic filler is not particularly limited and can be adjusted appropriately depending on the particle diameter, pore volume, specific surface area, and desired properties of the inorganic particle of the center portion of the (e) porous inorganic filler.

The (e) porous inorganic filler must be contained within a range of 1 % by mass or more and 15 % by mass or less, and more preferably within a range of 3 % by mass or more and 10 % by mass or less, based on the total dental resin-reinforced glass ionomer cement composition of the present invention. When the content of the (e) porous inorganic filler is less than 1 % by mass, the effect of reducing stringiness of the mixed product is not exhibited. On the other hand, when the content exceeds 15% by mass, the mechanical property is reduced, and the viscosity of the mixed product increases, resulting in poor mixability.

### <(f) Polymerization initiator>

The (f) polymerization initiator that can be used in the dental resin-reinforced glass ionomer cement composition of the present invention is not particularly limited, and any known polymerization initiator used in the dental field can be used without any limitation. As the type of the (f) polymerization initiator, there are those which initiate radical polymerization by the action of compounds of two or more components such as redox initiator (in the dental field, called sometimes as "chemical polymerization initiator", and hereinafter referred to as "chemical polymerization initiator".) and those which initiate radical polymerization by irradiation with light (photopolymerization initiators), and any of these polymerization initiators can be used in the present invention without any limitations. There are no particular limitations on the method for compounding the (f) polymerization initiator into the dental resin-reinforced glass ionomer cement composition of the present invention, as long as the (f) polymerization initiator can sufficiently polymerize the (d) polymerizable monomer. For example, when the dental resin-reinforced glass ionomer cement composition of the present invention consists of a powder material and a liquid material, or a first paste and a second paste, the (f) polymerization initiator may be compounded into both of them, or into either the powder material and the liquid material, or the first paste and the second paste. The dental resin-reinforced glass ionomer cement composition of the present invention may contain only a chemical polymerization initiator as the (f) polymerization initiator. The dental resin-reinforced glass ionomer cement composition of the present invention may not contain a chemical polymerization initiator as the (f) polymerization initiator. The dental resin-reinforced glass ionomer cement composition of the present invention may contain only a photopolymerization initiator as the (f) polymerization initiator. The dental resin-reinforced glass ionomer cement composition of the present invention may not contain a photopolymerization initiator as the (f) polymerization initiator. The dental resin-reinforced glass ionomer cement composition of the present invention may contain only a chemical polymerization initiator and a photopolymerization initiator as the (f) polymerization initiator. The dental resin-reinforced glass ionomer cement composition of the present invention may contain a chemical polymerization initiator and a photopolymerization initiator, as the (f) polymerization initiator.

Specific examples of the chemical polymerization initiator include peroxide/amine compound, peroxide/amine compound/aromatic sulfinic acid or its salt or aromatic sulfonyl compound, peroxide/amine compound/(thio) barbituric acid compound or (thio) barbiturate compound, peroxide/amine compound/borate compound, peroxide/ascorbic acid compound, peroxide/thiourea/vanadium compound or copper compound, (thio) barbituric acid compound/organometallic compound/organohalogen compound, a system combining aromatic sulfinic acid salts, borate compounds or (thio) barbiturate salts which initiate radical polymerization by the action of an acidic compound and an acidic compound, and an organic boron compound that initiates radical polymerization by reacting with oxygen or water, but are not limited thereto.

Examples of peroxides include sodium peroxodisulfate, potassium peroxodisulfate, ammonium peroxodisulfate, sodium peroxodiphosphate, potassium peroxodiphosphate, ammonium peroxodiphosphate, diacyl peroxides, alkyl peroxy esters, peroxydicarbonates, monoperoxycarbonates, peroxyketals, dialkyl peroxides, hydroperoxides and ketone peroxides. More specific examples include benzoyl peroxide, p-chlorobenzoyl peroxide, 2,4-dichlorobenzoyl peroxide, diacetyl peroxide, lauroyl peroxide, di-t-butyl peroxide, dicumyl peroxide, cumene hydroperoxide, t-butyl hydroperoxide, 1,1,3,3-tetramethylbutyl hydroperoxide, t-amyl hydroperoxide, iso-propylbenzene hydroperoxide, 5-phenyl-4-pentenyl hydroperoxide, t-butylperoxy isopropyl carbonate, methyl ethyl ketone peroxide, 1,1-bis (t-butylperoxy) cyclohexane, 1,1-bis (t-hexylperoxy) cyclohexane, and t-butylperoxybenzoate, but are not limited thereto.

The amine compound is preferably an aromatic secondary or aromatic tertiary amine, and specific examples thereof include N-methyl-p-toluidine, N-ethyl-p-toluidine, N-(2-hydroxyethyl)-p-toluidine, ethyl p-methyl aminobenzoate, N-methylaniline, N-ethylaniline, N-(2-hydroxyethyl) aniline, N,N-dimethylaniline, N,N-diethylaniline, N,N-bis (2-hydroxyethyl) aniline, N-(2-hydroxyethyl)-N-methylaniline, N-ethyl-N-(2-hydroxyethyl) aniline, N,N-dimethyl-p-toluidine, N,N-diethyl-p-toluidine, N,N-bis (2-hydroxyethyl)-p-toluidine and ethyl p-dimethyl aminobenzoate, but are not limited thereto.

Specific examples of an aromatic sulfinic acid or a salt thereof or an aromatic sulfonyl compound include benzenesulfinic acid, p-toluenesulfinic acid, o-toluenesulfinic acid, 2,4,6-trimethylbenzenesulfinic acid, 2,4,6-triisopropylbenzenesulfinic acid, and its sodium salt, potassium salt, lithium salt or ammonium salt, benzenesulfonyl chloride, benzenesulfonyl fluoride, benzenesulfonamide, benzenesulfonyl hydrazide, p-toluenesulfonyl chloride, p-toluenesulfonyl fluoride, p-toluenesulfonamide and p-toluenesulfonyl hydrazide, but are not limited thereto.

Specific examples of a (thio) barbituric acid compound or a (thio) barbituric acid salt compound include barbituric acid, 1,3-dimethylbarbituric acid, 1,3-diphenylbarbituric acid, 1,5-dimethylbarbituric acid, 5-butyl barbituric acid, 5-ethyl barbituric acid, 5-isopropyl barbituric acid, 5-cyclohexyl barbituric acid, 5-lauryl barbituric acid, 1,3,5-trimethyl barbituric acid, 1,3-dimethyl-5-ethyl barbituric acid, 1,3-dimethyl-n-butyl barbituric acid, 1,3-dimethyl-5-isobutyl barbituric acid, 1,3-dimethyl-5-cyclohexyl barbituric acid, 1,3-dimethyl-5-phenyl barbituric acid, 1-cyclohexyl-5-ethyl barbituric acid, 1-phenyl-5-benzyl barbituric acid, 1-benzyl-5-phenyl barbituric acid, thiobarbituric acid, 1,3-dimethylthiobarbituric acid, 5-phenylthiobarbituric acid and its alkali metal salts (lithium, sodium, potassium salts and the like), alkaline earth metal salts (calcium, strontium, barium salts and the like), ammonium salts, tetramethylammonium salts and tetraethylammonium salts, but are not limited thereto.

Examples of the borate compound include sodium salt, potassium salt, lithium salt, magnesium salt, tetramethylammonium salt, tetraethylammonium salt, tetrabutylammonium salt, methylpyridinium salt, ethylpyridinium salt, methylquinolinium salt and ethylquinolinium salt of trialkylphenylboron, trialkyl(p-chlorophenyl)boron, trialkyl(p-fluorophenyl)boron, trialkyl(p-butylphenyl)boron, trialkyl(p-butyloxyphenyl)boron, monoalkyltriphenylboron, monoalkyltris(p-chlorophenyl)boron, monoalkyltris(p-fluorophenyl)boron, monoalkyltris(p-butylphenyl)boron, monoalkyltris(p-butyloxyphenyl)boron, tetraphenylboron, tetrakis(p-chlorophenyl)boron, tetrakis(p-fluorophenyl)boron, tetrakis(p-butylphenyl)boron and tetrakis(p-butyloxyphenyl)boron (wherein the alkyl group is n-butyl group, n- octyl group, n-dodecyl group and the like) and the like, but are not limited thereto.

Specific examples of ascorbic acid compounds include L(+)-ascorbic acid, isoascorbic acid, L(+)-sodium ascorbate, L(+)-potassium ascorbate, L(+)-calcium ascorbate and sodium isoascorbate, but are not limited thereto.

Specific examples of thiourea compounds include 1,3-dimethylthiourea, tetramethylthiourea, 1,1-diethylthiourea, 1,1,3,3-tetraethylthiourea, 1-allyl-2-thiourea, 1,3-diallylthiourea, 1,3-dibutylthiourea, 1,3-diphenyl-2-thiourea, 1,3-dicyclohexylthiourea, ethylenethiourea, N-methylthiourea, N-phenylthiourea, N-benzoylthiourea and N-acetylthiourea, but are not limited thereto.

Specific examples of vanadium compounds include vanadium acetylacetonate, vanadyl acetylacetonate, vanadyl stearate, vanadium naphthenate and vanadium benzoylacetonate, but are not limited thereto.

Specific examples of the copper compound include copper acetate, copper salicylate, copper gluconate, copper oleate, copper benzoate, acetylacetone copper and copper naphthenate, but are not limited thereto.

Specific examples of the organometallic compound include, in addition to the above copper compound, manganese acetylacetone, manganese naphthenate, manganese octylate, cobalt acetylacetone, cobalt naphthenate, lithium acetylacetone, lithium acetate, zinc acetylacetone, zinc naphthenate, nickel acetylacetone, nickel acetate, aluminum acetylacetone, calcium acetylacetone, iron acetylacetone, chromium acetylacetone and sodium naphthenate, but are not limited to.

Specific examples of the organic halogen compound include tetramethyl ammonium chloride, tetraethyl ammonium chloride, trioctylmethyl ammonium chloride, dilauryl dimethyl ammonium chloride, lauryl dimethyl benzyl ammonium chloride, tetra-n-butyl ammonium chloride, benzyl dimethyl cetyl ammonium chloride and benzyl dimethyl stearyl ammonium chloride, but are not limited thereto.

As the acidic compound to be reacted with the aromatic sulfinates, borate compounds or (thio) barbiturates, any of an inorganic acid and an organic acid can be used without any limitation, but it is preferable to use an organic acid. Furthermore, from the viewpoint of the stability of the set product, it is most preferable to use an organic acid having a polymerizable group, that is, an acidic group-containing polymerizable monomer, as the organic acid. As the acidic group-containing polymerizable monomer, the type and number of polymerizable groups and acidic groups are not particularly limited, and an acidic group-containing polymerizable monomer commonly used in the dental field can be used. Examples of the acidic group include a carboxy group, a phosphoric acid group, a phosphonic acid group and a sulfonic acid group. Representative examples of the acidic group-containing polymerizable monomer used in the dental field include 10-(meth) acryloyloxydecyl dihydrogenphosphate, 6-(meth) acryloyloxyhexyl-3-phosphonoacetate, 4-(meth) acryloyloxyethyl trimellitic acid and its anhydride and 10-(meth) acryloyloxy-1,1-decanedicarboxylic acid, but are not limited thereto. These acidic compounds may be used alone or in a combination of a plurality thereof.

Specific examples of the organic boron compound that initiates polymerization by reacting with oxygen or water include trialkylborons such as triethylboron, tri (n-propyl) boron, triisopropylboron, tri (n-butyl) boron, tri (s-butyl) boron, triisobutylboron, tripentylboron, trihexylboron and tricyclohexylboron, as well as partial oxides thereof, but are not limited thereto.

Examples of the photopolymerization initiator includes photosensitizer and photosensitizer/photopolymerization promotor. Specific examples of the photosensitizer include α-diketones such as benzil, camphorquinone, α-naphtil, acetonaphtone, p,p'-dimethoxybenzil, p,p'-dichlorobenzylacetyl, pentadione, 1,2-phenanthrenquinone, 1,4-phenanthrenquinone, 3,4-phenanthrenquinone, 9,10-phenanthrenquinone and naphthoquinone; benzoin alkyl ethers such as benzoin, benzoin methyl ether and benzoin ethyl ether; thioxanthones such as thioxanthone, 2-chlorothioxanthone, 2-methylthioxanthone, 2-isopropylthioxanthone, 2-methoxythioxanthone, 2-hydroxythioxanthone, 2,4-diethylthioxanthone and 2,4-diisopropylthioxanthone; benzophenones such as benzophenone, p-chlorobenzophenone and p-methoxybenzophenone; acylphosphineoxides such as 2,4,6-trimethylbenzoyl diphenylphosphineoxide, bis (2,4,6-trimethylbenzoyl) phenylphosphine oxide, 2,6-dimethoxybenzoyl diphenylphosphine oxide and bis (2,6-dimethoxybenzoyl) phenylphosphine oxide; α-aminoacetophenones such as 2-benzyl-dimethylamino-1-(4-morpholinophenyl)-butanone-1,2-benzyl-diethyl-amino-1-(4-morpholinophenyl) propanone-1; ketals such as benzyldimethylketal, benzyldiethylketal and benzyl (2-methoxyethylketal); coumarins such as 3-(4-methoxybenzoyl) coumarin, 3-benzoyl-5,7-dimethoxycoumarin, 3,3'-carbonyl bis (7-diethylaminocoumarin) and 3,3'-carbonyl bis (7-dibutylaminocoumarin); and titanocenes such as bis (cyclopentadienyl)-bis [2,6-difluoro-3-(1-pyrolyl) phenyl] titanium, bis (cyclopentadienyl)-bis (pentanefluorophenyl) titanium and bis (cyclopentadienyl)-bis (2,3,5,6-tetrafluoro-4-disiloxyphenyl)-titanium, but are not limited thereto.

Specific examples of the photopolymerization promotors include tertiary amines such as N,N-dimethylaniline, N,N-diethylaniline, N,N-di-n-butylaniline, N,N-dibenzylaniline, N,N-dimethyl-p-toluidine, N,N-dimethyl-m-toluidine, N,N-diethyl-p-toluidine, p-bromo-N,N-dimethylaniline, m-chloro-N,N-dimethylaniline, p-dimethylamino benzaldehyde, p-dimethylamino acetophenone, p-dimethylamino benzoic acid, ethyl p-dimethylaminobenzoate, isoamyl p-dimethylaminobenzoate, N,N-dimethylanthranilic acid methyl ester, N,N-dihydroxyethylaniline, N,N-dihydroxyethyl-p-toluidine, p-dimethylaminophenyl alcohol, p-dimethylaminostyrene, N,N-dimethyl-3,5-xylidine, 4-dimethylaminopyridine, N,N-dimethyl-α-naphthylamine, N,N-dimethyl-β-naphthylamine, triethanolamine, tributylamine, tripropylamine, triethylamine, N-methyldiethanolamine, N-ethyldiethanolamine, N,N-dimethylhexylamine, N,N-dimethyldodecylamine, N,N-dimethylstearylamine, N,N-dimethylaminoethyl (meth)acrylate, N,N-diethylaminoethyl (meth)acrylate and 2,2'-(n-butyl imino) diethanol; secondary amines such as N-phenylglycine; barbiturates such as 5-butylbarbituric acid, 1-benzyl-5-phenylbarbituric acid, 1,3,5-trimethylbarbituric acid, sodium 1,3,5-trimethylbarbiturate and calcium 1,3,5- trimethylbarbiturate; triazine compounds such as 2,4,6-tris (trichloromethyl)-s-triazine, 2-phenyl-4,6-bis (trichloromethyl)-s-triazine, 2-(p-chlorophenyl)-4,6-bis (trichloromethyl)-s-triazine and 2-(4-biphenyl)-4,6-bis (trichloromethyl)-s-triazine; diaryliodonium salts such as diphenyliodonium chloride, diphenyliodonium hexafluorophosphate, diphenyliodonium hexafluoroantimonate, diphenyliodonium tetrafluoroborate, diphenyliodonium tetrakis (pentafluorophenyl) borate, bis-(4-methylphenyl) iodonium hexafluorophosphate, bis-(4-methylphenyl) iodonium hexafluoroantimonate, bis-(4-methylphenyl) iodonium tetrakis (pentafluorophenyl) borate, bis (4-tert-butylphenyl) iodonium hexafluoroantimonate, and bis (4-tert-butylphenyl) iodonium tetrakis (pentafluorophenyl) borate; tin compounds such as dibutyltin diacetate, dibutyltin dilaurate, dioctyltin dilaurate, dioctyltin diversate, dioctyltin bis (mercaptoacetic acid isooctyl ester) salt and tetramethyl-1,3-diacetoxydistannoxane; aldehyde compounds such as lauryl aldehyde and terephthalaldehyde; and sulfur-containing compounds such as dodecyl mercaptan, 2-mercaptobenzoxazole, 1-decanethiol and thiosalicylic acid, but are not limited thereto.

In order to enhance photopolymerization promotion performance, it is effective to add, in addition to the above photopolymerization promoter, oxycarboxylic acids such as citric acid, malic acid, tartaric acid, glycolic acid, gluconic acid, α-oxyisobutyric acid, 2-hydroxypropanoic acid, 3-hydroxypropanoic acid, 3-hydroxybutanoic acid, 4-hydroxybutanoic acid and dimethylolpropionic acid, but are not limited thereto.

In addition, these of the (f) polymerization initiator can be used not only singly but also in a combination of a plurality kinds thereof, regardless of the polymerization manner or the polymerization method. Furthermore, there is no problem even if these of the(f) polymerization initiator are subjected to a secondary treatment such as encapsulation in a microcapsule, if necessary.

It is preferable that a content of the (f) polymerization initiator is 0.01 % by mass or more and 10 % by mass ore less and more preferably 0.1 % by mass or more and 5 % by mass or less based on the whole of the dental resin-reinforced glass ionomer cement composition of the present invention. When the content of the (f) polymerization initiator is less than 0.01 % by mass, there is a case where the settability deteriorates and the mechanical characteristic decreases. When the content of the (f) polymerization initiator exceeds 10 % by mass, there is a case where the storage stability deteriorates.

### <(g) Organic-inorganic composite filler>

In order to further improve the mixability while maintaining the effect of reducing the stringiness of the mixed material by the (e) porous inorganic filler, it is preferable that the dental resin-reinforced glass ionomer cement composition of the present invention further comprise (g) organic-inorganic composite filler. The (g) organic-inorganic composite filler that can be used in the dental resin-reinforced glass ionomer cement composition of the present invention is a composite particle consisting of an organic portion where a polymerizable monomer is cured and an inorganic portion contained in the form of an inorganic filler, and the inorganic filler exists in a dispersed state in the cured polymerizable monomer. The (g) organic-inorganic composite filler can be obtained by making a polymerizable monomer containing a polymerization initiator, and an inorganic filler in as homogeneous a state as possible, curing the polymerizable monomer, and pulverizing the cured product as necessary.

The molecular structure of the polymerizable monomer that can be used to manufacture the (g) organic-inorganic composite filler is not particularly limited, and the same polymerizable monomer as the above described (d) polymerizable monomer can be used.

It is preferable that a content of the polymerizable monomer contained in the raw material of the (g) organic-inorganic composite filler is 15 % by mass or more and 90 % by mass or less and more preferably 15 % by mass or more and 60 % by mass or less. When the content of the polymerizable monomer in the (g) organic-inorganic composite filler is less than 15 % by mass, there is a case where the dispersion of the inorganic filler in the organic-inorganic composite filler is insufficient and the mechanical characteristic of the organic-inorganic composite filler itself decreases. On the other hand, when the content of the polymerizable monomer exceeds 90 % by mass, there is a case where the abrasion resistance of the dental resin-reinforced glass ionomer cement composition of the present invention decreases.

Next, a polymerization initiator that can be used in the manufacture of the (g) organic-inorganic composite filler will be described. The polymerization initiator is not particularly limited, and known polymerization initiators such as a photopolymerization initiator, a chemical polymerization initiator and a thermal polymerization initiator can be used. Among these, it is preferable to use a thermal polymerization initiator since it is excellent in production efficiency of the (g) organic-inorganic composite filler. As the thermal polymerization initiator, an organic peroxides such as benzoyl peroxide, an azo compound such as azobisisobutyronitrile and the like may be suitably used. These polymerization initiators can be used not only singly but also in a combination of plurality thereof, regardless of the polymerization manner or the polymerization method. The amount of the polymerization initiator to be added is not particularly limited, but is generally 0.1 % by mass to 10 % by mass based on 100 % by mass of all polymerizable monomers used in the production of the (g) organic-inorganic composite filler.

Next, an inorganic filler that can be used in the manufacture of the (g) organic-inorganic composite filler will be described. The inorganic filler is not particularly limited in terms of its constituent elements, and any known inorganic filler can be used. Specific examples of the inorganic filler include inorganic oxides such as silica, alumina, titania, zirconia, strontium oxide, barium oxide, yttrium oxide, lanthanum oxide and ytterbium oxide; inorganic complex oxides such as silica-zirconia, silica-titania, silica-titania-barium oxide and silica-titania-zirconia; glasses such as molten silica, quartz, aluminosilicate glass, fluoroaluminosilicate glass, borosilicate glass, alminoborate glass and boroaluminosilicate glass; and metallic fluorides such as calcium fluoride, barium fluoride, strontium fluoride, yttrium fluoride, lanthanum fluoride and ytterbium fluoride.

A shape of these of the inorganic filler is not particularly limited, and may be any shape such as spherical, needle-like, plate-like, ground-like and scaly-shapes, and aggregate thereof may be used without any problems. The above described inorganic filler is not limited to these and may be used alone or in a combination of plurality thereof.

There are no particular limitations on the particle diameter of the inorganic filler, but in consideration of the balance of various properties in the dental resin-reinforced glass ionomer cement composition of the present invention, it is preferable that the 50% particle diameter (D50) is 0.005 µm or more and 3 µm or less. When the 50% particle diameter (D50) of the inorganic filler is less than 0.005 µm, there is a case where it is impossible to highly fill the inorganic filler into the (g) organic-inorganic composite filler and the abrasion resistance of the dental resin-reinforced glass ionomer cement composition of the present invention decreases. Furthermore, when the 50% particle diameter (D50) of the inorganic filler exceeds 3 µm, there is a case where the polishing property of the dental resin-reinforced glass ionomer cement composition of the present invention decreases and a smooth surface is not obtained. In the dental resin-reinforced glass ionomer cement composition of the present invention, the inorganic filler constituting the (g) organic-inorganic composite filler may consist of only an inorganic filler having a 50% particle diameter (D50) of 0.005 µm or more and 3 µm or less.

It is preferable that these inorganic fillers are subjected to a surface treatment to be made hydrophobic. This surface treatment enables high filling of the inorganic filler in the organic-inorganic composite filler to improve the mechanical characteristic of the organic-inorganic composite filler itself. The surface treatment agent that can be used for the surface treatment of the inorganic filler is not particularly limited, and known agents such as an organosilicon compound, an organozirconium compound, an organotitanium compound and an organoaluminum compound can be used, but the most commonly used is an organosilicon compound. Specific examples of the organosilicon compound include methyltrimethoxysilane, ethyltrimethoxysilane, methoxytripropylsilane, propyltriethoxysilane, hexyltrimethoxysilane, vinyltrimethoxysilane, vinyltriethoxysilane, vinyltrichlorosilane, vinyltri (β-methoxyethoxy) silane, 3-(meth) acryloyloxypropyl trimethoxysilane, 8-(meth) acryloyloxyoctyl trimethoxysilane, 3-glycidoxypropyl trimethoxysilane, 3-mercaptopropyl trimethoxysilane, 3-aminopropyl trimethoxysilane, 3-aminopropyl triethoxysilane, methyltrichlorosilane, phenyltrichlorosilane, trimethylsilylisocyanate, vinylsilyltriisocyanate, phenylsilyltriisocyanate and hexamethyldisilazane, but are not limited thereto. These surface treatment agents can be used alone or in a combination of a plurality thereof. The method of surface treatment is not particularly limited, and any known method can be applied. Furthermore, the amount of the surface treatment agent relative to the inorganic filler when performing the surface treatment is not particularly limited, and may be appropriately adjusted depending on the particle diameter of the inorganic filler and the like.

It is preferable that a content of the inorganic filler contained in the raw material of the (g) organic-inorganic composite filler is preferably 10 % by mass or more and 85 % by mass or less and more preferably 40 % by mass or more and 85 % by mass or less. When the content of the inorganic filler is less than 10 % by mass, there is a case where the abrasion resistance of the dental resin-reinforced glass ionomer cement composition of the present invention decreases. On the other hand, when the content of the inorganic filler exceeds 85 % by mass, there is a case where the dispersion of the inorganic filler in the organic-inorganic composite filler is insufficient and the mechanical characteristic of the organic-inorganic composite filler it self decreases. The (g) organic-inorganic composite filler used in the dental resin-reinforced glass ionomer cement composition of the present invention may contain 10 % by mass or more and 85 % by mass or less of an inorganic filler having a 50% particle diameter (D50) of 0.05 µm or more and 3 µm or less. The dental resin-reinforced glass ionomer cement composition of the present invention may contain only an organic-inorganic composite filler in which the content of the inorganic filler contained in the raw material is 10 % by mass or more and 85 % by mass or less, as the (g) organic-inorganic composite filler. The dental resin-reinforced glass ionomer cement composition of the present invention may contain only an organic-inorganic composite filler containing 10 % by mass or more and 85 % by mass or less of an inorganic filler having a 50% particle diameter (D50) of 0.005 µm or more and 3 µm or less, as the (g) organic-inorganic composite filler.

Next, the method for manufacturing the (f) organic-inorganic composite filler will be described by taking as an example a case in which a thermal polymerization initiator is used. The (f) organic-inorganic composite filler is prepared through the following main Step 1) to Step 4).
Step 1) a step of mixing the components constituting the (e1) organic-inorganic composite filler such as a polymerizable monomer, a thermal polymerization initiator and an inorganic filler to obtain a mixture.
Step 2) a step of applying heat to the mixture to polymerize the polymerizable monomer to obtain a cured product.
Step 3) a step of pulverizing the cured product as necessary to obtain an organic-inorganic composite filler.
Step 4) a step of performing a surface treatment on the organic-inorganic composite filler as necessary.

In the dental resin-reinforced glass ionomer cement composition of the present invention, the ground organic-inorganic composite filler obtained in Step 3) may be used as is, and the surface-treated organic-inorganic composite filler obtained in step 4) may be used. Furthermore, in a case in which it is not in the form of a lump but is already in the form of fine particles at the stage of Step 2), it may be used as it is as the organic-inorganic composite filler. Furthermore, the organic-inorganic composite filler may be used after subjecting a surface treatment in Step 4).

Examples of the step of obtaining a mixture of the components in Step 1) include a method of mixing the components such as a polymerizable monomer, a thermal polymerization initiator and an inorganic filler using a kneader, a method of aggregating an inorganic filler to obtain aggregated fillers having pores and a size of several µm to several tens of µm, immersing the aggregated fillers in a solution in which a thermal polymerization initiator and a polymerizable monomer are dissolved in an organic solvent to obtain a slurry, and then removing the organic solvent at a low temperature under reduced pressure to allow the polymerizable monomer to penetrate and cover the inside and surface of the aggregated filler, thereby mixing the components, and a method of press-molding an inorganic filler to obtain an inorganic filler molded body, immersing the molded body in a polymerizable monomer containing a thermal polymerization initiator and allowing the polymerizable monomer to penetrate into the inside of the molded body, thereby mixing the components, but are not limited thereto. In this step, by dissolving a surface treatment agent such as the above described organosilicon compound in the polymerizable monomer, the surface treatment of the inorganic filler and the mixing of the various components can be carried out simultaneously. This makes it possible to omit the step of surface treating the inorganic filler before mixing the various components.

In the step of obtaining a cured product in Step 2), the polymerization temperature and polymerization time may be appropriately adjusted depending on the properties of the used thermal polymerization initiator and based on the discoloration of the organic-inorganic composite filler due to heat and the amount of residual unpolymerized monomer, but the polymerization temperature is generally 70 °C or higher and 150 °C or lower, and the polymerization time is several minutes to several hours. Depending on the polymerization method, polymerization conditions can be appropriately selected, such as polymerization in air, polymerization in an inert gas atmosphere such as nitrogen or argon, polymerization under normal pressure, or polymerization under pressure.

In the step of obtaining the organic-inorganic composite filler by pulverization in Step 3), a pulverization method similar to that used in manufacturing the above described (a) acid-reactive glass powder can be used. There are no particular limitations on the 50% particle diameter (D50) of the (g) organic-inorganic composite filler, and it is possible to appropriately adjust according to the desired property to be imparted to the dental resin-reinforced glass ionomer cement composition of the present invention, but in order to effectively reduce stringiness of the mixed material in the dental resin-reinforced glass ionomer cement composition of the present invention, the 50% particle diameter (D50) is preferably 1 µm or more and 50 µm or less. When the 50% particle diameter (D50) of the (g) organic-inorganic composite filler is less than 1 µm, there is a case where the mixability decreases, and when the 50% particle diameter (D50) exceeds 50 µm, there is a case where the mechanical characteristic decreases. The dental resin-reinforced glass ionomer cement composition of the present invention may comprise only an organic-inorganic composite filler having a 50 % particle diameter (D50) within the range of 1 µm or more and 10 µm or less, as the (g) organic-inorganic composite filler.

In the step of subjecting the organic-inorganic composite filler to a surface treatment in Step 4), the same surface treatment agent as that which can be used for the surface treatment of the inorganic filler described above can be used. As for the surface treatment method, a known method can be applied, similar to the surface treatment of the inorganic filler. Furthermore, the amount of the surface treatment agent with respect to the organic-inorganic composite filler when performing the surface treatment is not particularly limited and may be appropriately adjusted depending on the particle diameter of the organic-inorganic composite filler and the like, but it is preferably 0.1 parts by mass or more and 5 parts by mass or less with respect to 100 parts by mass of the organic-inorganic composite filler.

When the dental resin-reinforced glass ionomer cement composition of the present invention comprises the (g) organic-inorganic composite filler, it is preferable to balance the content of the (e) porous inorganic filler. That is, it is preferable that a total content of the (e) porous inorganic filler and the (g) organic-inorganic composite filler is 2 % by mass or more and 20 % by mass or less based on the whole of the dental resin-reinforced glass ionomer cement composition, and the mass ratio of the content of the (e) porous inorganic filler to the content of the (g) organic-inorganic composite filler [(g) organic-inorganic composite filler/(e) porous inorganic filler] is 0.5 or more and 2.5 or less. By comprising the (e) porous inorganic filler and the (g) organic-inorganic composite filler within this range, it is possible to keep low viscosity of the mixed material to further improve mixability without adversely affecting other properties. Furthermore, the total content is more preferably 3% by mass or more and 15% by mass or less. Furthermore, the mass ratio is more preferably 0.8 or more and 2.0 or less.

When the total content is less than 2% by mass, there is a case where the effect of reducing stringiness of the mixed material is no sufficiently obtained. On the other hand, when the total content exceeds 20% by mass, there is a case where the mixing property, the mechanical property and transparency are reduced. Also, when the mass ratio is less than 0.5, there is a case where the viscosity of the mixed material cannot be kept low, and the mixing property is deteriorated. On the other hand, when the mass ratio exceeds 2.5, there is a case where the mechanical property and transparency are reduced.

### <Other component>

The dental glass ionomer cement composition of the present invention may arbitrarily contain an acidic compound to such a range that various properties are not adversely affected, for the purpose of adjusting the working time and set time. Specific examples of the acidic compound include carboxylic acid compounds such as tartaric acid, citric acid, maleic acid, fumaric acid, malic acid, oxalic acid, malonic acid, ascorbic acid, mesaconic acid, itaconic acid, oxaloacetic acid, glutaric acid, aconitic acid, tricarballylic acid, 1-butene-1,2,4-tricarboxylic acid and 3-butene-1,2,3-tricarboxylic acid; phosphoric acid compounds such as phosphoric acid, pyrophosphoric acid and tripolyphosphoric acid, but are not limited to thereto. Furthermore, it is possible to use these acidic compounds partially neutralized with a basic compound without any problems. As the basic compound, it is possible to use compound that is the same as the compound that can be used to neutralize the (b) polyalkenoic acid. These acidic compounds may be used alone or in a combination of several kinds thereof. When the acidic compound is contained in the dental resin-reinforced glass ionomer cement composition of the present invention, the content of the acidic compound is preferably 0.1 % by mass or more and 15 % by mass or less based on the whole of the composition. Further, the dental resin-reinforced glass ionomer cement composition of the present invention may not contain an acidic compound.

Further, a surfactant can be optionally contained in the dental resin-reinforced glass ionomer cement composition of the present invention to such a range that various properties are not adversely affected, for the purpose of adjusting the initial compatibility of the powder material and liquid material, or first paste and second paste or mixed material property. The surfactant which can be used in the dental resin-reinforced glass ionomer cement composition of the present invention may be any of an ionic surfactant and a nonionic surfactant.

Specific examples of the anionic surfactant in the ionic surfactant include aliphatic carboxylic acid metal salts such as sodium stearate, sulfated aliphatic carboxylic acid metal salts such as sodium dioctyl sulfosuccinate and metal salts of higher alcohol sulfate ester such as sodium stearyl sulfate. In addition, examples of the cationic surfactant include an adduct of higher alkylamine and ethylene oxide, amines made from lower amine, and alkyltrimethylammonium salts such as lauryltrimethylammoniun chloride. Further, examples of the amphoteric surfactant include metal salts of higher alkylaminopropionic acid such as sodium stearylaminopropionate, and betaines such as lauryldimethylbetaine.

Examples of the nonionic surfactant include polyethylene glycol type and polypropylene glycol type in which ethylene oxide or propylene oxide is added to higher alcohols, alkyl phenols, fatty acids, higher fatty amines or aliphatic amides, and polyhydric alcohol type in which polyhydric alcohols, diethanolamines or saccharides is ester bonded to a fatty acid.

The above described surfactant is not limited to these, and may be used alone or in a combination of a few kinds thereof. When the surfactant is contained in the dental resin-reinforced glass ionomer cement composition of the present invention, it is preferable that the content of the surfactant is 0.001 % by mass or more and 5 % by mass or less based on the whole of the composition. The dental resin-reinforced glass ionomer cement composition of the present invention may not contain a surfactant.

Further, a non acid-reactive powder having no pore can be arbitrarily contained in the dental resin-reinforced glass ionomer cement composition of the present invention to such a range that various properties are not adversely affected, for the purpose of adjusting operability, a mechanical characteristic , or a curing characteristic.

As the non acid-reactive powder having no pore used in the dental resin-reinforced glass ionomer cement composition of the present invention, any non acid-reactive powder as long as the non acid-reactive powder does not contain element which may react with an acid group of the polyalkenic acid can be used without any limitation. Examples of the non acid-reactive powder having no pore include known powder in the dental field such as an inorganic filler and an organic filler, and these can be used alone or in a combination of a few of them. Among them, it is especially preferable that an inorganic filler is used. In addition, a shape of the non acid-reactive powder having no pore is not particularly limited, and may be any shape such as arbitral shapes such as spherical, needle-like, plate-like, ground-like and scaly-shapes may be used. There is no particular limitation on the 50% particle diameter (D50) of these of the non acid-reactive powder having no pore, but it is preferably 0.001 µm or more and 30 µm or less.

Specific examples of the inorganic filler having no pore include quartz, amorphous silica, ultrafine silica, various glasses which does not contain element which may react with an acid group (including a glass by melting method, synthetic glass by sol-gel method, a glass produced by a vapor phase reaction, and the like), silicon nitride, silicon carbide, boron carbide and the like, but is not limited thereto. The inorganic filler having no pore can be used alone or in a combination of a few kinds.

When the non acid-reactive powder having no pore is contained in the dental resin-reinforced glass ionomer cement composition of the present invention, it is preferable that the content of the non acid-reactive powder having no pore is within a range of 0.001 % by mass or more and 20% by mass or less based on the whole composition. The dental resin-reinforced glass ionomer cement composition of the present invention may not contain a non acid-reactive powder having no pore.

Furthermore, when the dental resin-reinforced glass ionomer cement composition for luting of the present invention includes a paste form, a thickener can be optionally contained to such a range that various properties are not adversely affected, for the purpose of adjusting the paste property.

As the thickener which can be used in the dental resin-reinforced glass ionomer cement composition of the present invention, any of an inorganic thickener and an organic thickener may be used. Specific examples of an inorganic thickener include fumed silica, calcium carbonate, calcium silicate, magnesium silicate, and a clay mineral such as saponite, montmorillonite, beidellite, vermiculite, sauconite, stevensite, hectorite, smectite, nekutaito and sepiolite.

Specific examples of an organic thickener include methyl cellulose, hydroxyethyl cellulose, hydroxymethyl cellulose, hydroxypropyl cellulose, carboxymethyl cellulose, sodium carboxymethyl cellulose, calcium carboxymethyl cellulose, carboxypolymethylene, sodium alginate, propylene glycol alginate ester, sodium polyacrylate, starch, starch sodium glycolate, starch phosphate ester, polyvinyl pyrrolidone, carboxyvinyl polymer, khaya gum, arabic gum, karaya gum and guar gum and xanthan gum.

The above described thickener is not limited to these, and may be used alone or in a combination of a few kinds thereof. When the thickener is contained in the dental resin-reinforced glass ionomer cement composition of the present invention, it is preferable that the content of the thickener in the paste is 0.001 % by mass or more and 10.0 % by mass or less. The dental resin-reinforced glass ionomer cement composition of the present invention may not contain a thickener.

In addition, the dental resin-reinforced glass ionomer cement composition of the present invention may arbitrarily contain a polymerization inhibitor, a chain transfer agent, a discoloration inhibitor, an ultraviolet absorber, a preservative, an antibacterial agent, a colorant, a fluorescent agent, an inorganic fiber material, an organic fiber material and other conventionally known additives, as necessary.

The dental resin-reinforced glass ionomer cement composition of the present invention may be provided in any form, such as powder material/liquid material, paste/paste, paste/powder material or paste/liquid material, so long as the (a) acid-reactive glass powder does not coexist with the (b) polyalkenoic acid in the presence of the (c) water.

It is preferable that the dental resin-reinforced glass ionomer cement composition of the present invention shortens the time until shaping can begin by adjusting the compound amounts of the above described components, thereby shortening treatment time. In order to shorten the treatment time, the shaping start time is preferably within 40 seconds, and more preferably within 30 seconds and further preferably within 20 seconds.

The dental resin-reinforced glass ionomer cement composition of the present invention can be used in a wide range of applications in dental treatment, such as pit and fissure sealant, lining material, abutment construction material and tooth surface coating material, in addition to being used as filling material and luting materials.

### [Example]

Hereinafter, the present invention will be described in detail with reference to Examples and Comparative Examples. However, the present invention is not limited to these Examples. The components (a) to (g) and other components used for manufacturing the dental resin-reinforced glass ionomer cement compositions of Examples and Comparative Examples, their abbreviations and preparing methods are as follows.

### [(a) Acid-reactive glass powder]

·G1: acid-reactive glass powder 1 (G1) (fluoroaluminosilicate glass powder, 50% particle diameter (D50): 2.5 µm)
·G2: acid-reactive glass powder 2 (G2) (silane-treated fluoroaluminosilicate glass powder, 50% particle diameter (D50): 2.5 µm)
·G3: acid-reactive glass powder 3 (G3) (silane-treated fluoroaluminosilicate glass powder, 50% particle diameter (D50): 30 µm)
·G4: acid-reactive glass powder 4 (G4) (silane-treated fluoroaluminosilicate glass powder, 50% particle diameter (D50): 0.45 µm)

### [(b) Polyalkenoic acid]

·PCA1: acrylic acid homopolymer powder (weight average molecular weight: 50,000)
·PCA2: acrylic acid homopolymer powder (weight average molecular weight: 15,000)
·PCA3: acrylic acid homopolymer powder (weight average molecular weight: 350,000)

### [(c) Water]

·IEW: Ion-exchanged water

### [(d) Polymerizable monomer]

·GDMA: glycerol-1,3-dimethacrylate (hydroxyl group-containing polymerizable monomer)
·HEMA: 2-hydroxyethyl methacrylate (hydroxyl group-containing polymerizable monomer)
·4-AET: 4-acryloxyethyl trimellitic acid (acidic group-containing polymerizable monomer)
·FAM: tetrafunctional acrylamide monomer ("FOM-03006" (manufactured by Fujifilm Wako Pure Chemical Industries, Ltd.))
·Bis-GMA: bisphenol A diglycidyl methacrylate (hydroxyl group-containing polymerizable monomer)

### [(e) Porpus inorganic filler]

PIF1: Porous inorganic filler (SiO₂: 80 % by mass, ZrO₂: 20 % by mass, 50 % particle diameter (D50): 2.0 µm, pore volume: 0.08 cc/g, specific surface area: 15 m²/g)
PIF2: Porous inorganic filler (SiO₂: 80 % by mass, ZrO₂: 20 % by mass, 50 % particle diameter (D50): 3.0 µm, pore volume: 0.20 cc/g, specific surface area: 135 m²/g)
PIF3: Porous inorganic filler (SiO₂: 80 % by mass, ZrO₂: 20 % by mass, 50 % particle diameter (D50): 4.0 µm, pore volume: 0.30 cc/g, specific surface area: 200 m²/g)
PIF4: Porous inorganic filler (SiO₂: 100 % by mass, 50% particle diameter (D50): 9.0 µm, pore volume: 0.80 cc/g, specific surface area: 200 m²/g)
PIF5: Surface-treated porous inorganic filler (SiO₂: 80 % by mass, ZrO₂: 20 % by mass, 50 % particle diameter (D50): 3.0 µm, pore volume: 0.19 cc/g, specific surface area: 133 m²/g)
PIF6: Porous inorganic filler (SiO₂: 94 % by mass, TiO₂: 6 % by mass, 50 % particle diameter (D50): 2.9 µm, pore volume: 0.21 cc/g, specific surface area: 173 m²/g)
PIF7: Porous inorganic filler (SiO₂: 70 % by mass, ZrO₂: 30 % by mass, 50 % particle diameter (D50): 2.9 µm, pore volume: 0.19 cc/g, specific surface area: 161 m²/g)
PIF8: Porous inorganic filler (SiO₂: 65 % by mass, ZrO₂: 35 % by mass, 50 % particle diameter (D50): 2.9 µm, pore volume: 0.17 cc/g, specific surface area: 149 m²/g)

### [(f) Polymerization initiator]

·KPS: potassium peroxodisulfate
·TSNa: sodium p-toluenesulfinate
·AA: L(+)-ascorbic acid
·CQ: dl-camphorquinone

### [(g) Organic-inorganic composite filler]

·O1: organic-inorganic composite filler 1 (50% particle diameter (D50): 30 µm, content of inorganic filler: 50 % by mass, primary average particle diameter of inorganic filler: 16 nm)
·O2: organic-inorganic composite filler 2 (50% particle diameter (D50): 25 µm, content of inorganic filler: 25 % by mass, primary average particle diameter of inorganic filler: 16 nm,)
·O3: organic-inorganic composite filler 3 (50% particle diameter (D50): 20 µm, content of inorganic filler: 50 % by mass, 50% particle diameter (D50) of inorganic filler: 0.5 µm,)
·O4: organic-inorganic composite filler 4 (50% particle diameter (D50): 55 µm, content of inorganic filler: 75 % by mass, 50% particle diameter (D50) of inorganic filler: 4 µm,)

### [Others]

·Fuselex X: Crushed silica filler (non acid-reactive powder having no pore, 50% particle diameter (D50): 3.0 µm, pore volume: less than 0.01 cc/g)
·Aerosil R972: Spherical silica filler (thickener, primary particle diameter 16 nm, pore volume: less than 0.01 cc/g)

### [Manufacture of (a) acid-reactive glass powder]

### [Manufacture of acid-reactive glass powder 1 (G1)]

Various raw materials: silica, alumina, aluminum phosphate, sodium fluoride, and strontium carbonate (glass composition: SiO₂: 26.4 % by mass, Al₂O₃: 29.3 % by mass, SrO: 20.5 % by mass, P₂O₅: 10.9 % by mass, Na₂O: 2.5 % by mass, and F: 10.4 % by mass) were mixed and the mixed material was molten at 1400 °C in a melting furnace. The molten liquid was taken out from the melting furnace and was quenched in water to manufacture a fluoroaluminosilicate glass. The resulting fluoroaluminosilicate glass was pulverized until the 50% particle diameter (D50) became 2.5 µm to obtain acid-reactive glass powder 1 (G1). The 50% particle diameter was measured by a laser diffraction/scattering type grain size distribution measuring apparatus (Microtrac MT3300EXII, manufactured by Microtrac Bell Co., Ltd.).

### [Manufacture of acid-reactive glass powder 2 (G2)]

A surface treatment liquid (total mass: 9.2 parts by mass) was prepared by mixing 1.0 part by mass of 3-methacryloyloxypropyl trimethoxysilane, 0.1 part by mass of ion-exchanged water and 8.1 parts by mass of absolute ethanol. This surface treatment liquid and 100 parts by mass of acid-reactive glass powder 1 (G1) were dry-mixed, and then heat-treated at 110 °C for 5 hours using a hot air dryer to obtain acid-reactive glass powder 2 (G2).

### [Manufacture of acid-reactive glass powder 3 (G3)]

A fluoroaluminosilicate glass was obtained in the same manner as in the manufacture of acid-reactive glass powder 1 (G1). The obtained fluoroaluminosilicate glass was pulverized until the 50% particle diameter (D50) became 30 µm to obtain an acid-reactive glass powder. The obtained fluoroaluminosilicate glass was pulverized until the 50% particle diameter (D50) became 30 µm to obtain an acid-reactive glass powder. The 50% particle diameter was measured by a laser diffraction/scattering type grain size distribution measuring apparatus (Microtrac MT3300EXII, manufactured by Microtrac Bell Co., Ltd.). A surface treatment liquid (total mass: 9.2 parts by mass) was prepared by mixing 1.0 part by mass of 3-methacryloyloxypropyl trimethoxysilane, 0.1 part by mass of ion-exchanged water and 8.1 parts by mass of absolute ethanol. This surface treatment liquid and 100 parts by mass of the above described acid-reactive glass powder were dry-mixed, and then heat-treated at 110 °C for 5 hours using a hot air dryer to obtain acid-reactive glass powder 3 (G3).

### [Manufacture of acid-reactive glass powder 4 (G4)]

A fluoroaluminosilicate glass was obtained in the same manner as in the manufacture of the acid-reactive glass powder 1 (G1). The obtained fluoroaluminosilicate glass was pulverized until the 50% particle diameter (D50) became 0.45 µm to obtain an acid-reactive glass powder. The 50% particle diameter was measured by a laser diffraction/scattering type grain size distribution measuring apparatus (Microtrac MT3300EXII, manufactured by Microtrac Bell Co., Ltd.). A surface treatment liquid (total mass: 18.3 parts by mass) was prepared by mixing 3.0 part by mass of 3-methacryloyloxypropyl trimethoxysilane, 0.3 part by mass of ion-exchanged water and 15.0 parts by mass of absolute ethanol. This surface treatment liquid and 100 parts by mass of the above described acid-reactive glass powder were dry-mixed, and then heat-treated at 110 °C for 5 hours using a hot air dryer to obtain acid-reactive glass powder 4 (G4).

### [Manufacture of surface-treated porous inorganic filler (PIF5)]

A surface treatment liquid (total mass: 9.2 parts by mass) was prepared by mixing 0.3 part by mass of 3-methacryloyloxypropyl trimethoxysilane, 0.1 part by mass of ion-exchanged water and 8.8 parts by mass of absolute ethanol. This surface treatment liquid and 100 parts by mass of Porous inorganic filler (PIF2) were dry-mixed, and then heat-treated at 110 °C for 5 hours using a hot air dryer to obtain Porous inorganic filler (PIF5).

### [Manufacture of organic-inorganic composite filler 1 (O1)]

A resin mixture was prepared by mixing 50 parts by mass of urethane dimethacrylate (hereinafter, UDMA), 50 parts by mass of neopentyl glycol dimethacrylate and 0.1 parts by mass of benzoyl peroxide (hereinafter, BPO). After kneading 50 parts by mass of the resin mixture and 50 parts by mass of Aerosil R972 until homogeneous, the kneaded material was heated at 100 °C for 4 hours in a nitrogen atmosphere to obtain a cured product. The obtained cured product was pulverized until the 50% particle diameter (D50) became 30 µm to obtain the organic-inorganic composite filler 1 (O1). The 50% particle diameter was measured by a laser diffraction/scattering type grain size distribution measuring apparatus (Microtrac MT3300EXII, manufactured by Microtrac Bell Co., Ltd.).

### [Manufacture of organic-inorganic composite filler 2 (O2)]

A resin mixture was prepared by mixing 75 parts by mass of UDMA, 25 parts by mass of ethylene glycol dimethacrylate and 0.2 parts by mass of BPO. After kneading 75 parts by mass of the resin mixture and 25 parts by mass of Aerosil R972 until homogeneous, the kneaded material was heated at 100 °C for 4 hours in a nitrogen atmosphere to obtain a cured product. The obtained cured product was pulverized until the 50% particle diameter (D50) became 250 µm to obtain the organic-inorganic composite filler 2 (O2). The 50% particle diameter was measured by a laser diffraction/scattering type grain size distribution measuring apparatus (Microtrac MT3300EXII, manufactured by Microtrac Bell Co., Ltd.).

### [Manufacture of organic-inorganic composite filler 3 (O3)]

A surface treatment liquid (total mass: 17.0 parts by mass) was prepared by mixing 6.0 part by mass of 3-methacryloyloxypropyl trimethoxysilane, 1.0 part by mass of ion-exchanged water and 10.0 parts by mass of absolute ethanol. In addition, fluoroaluminosilicate glass was prepared in the same manner as in the manufacture of the acid-reactive glass powder 1 (G1). The obtained fluoroaluminosilicate glass was pulverized until the 50% particle diameter (D50) became 0.5 µm to obtain a fluoroaluminosilicate glass powder. This surface treatment liquid and 100 parts by mass of the above described fluoroaluminosilicate glass powder (50% particle diameter (D50): 0.5 µm) were dry-mixed, and then heat-treated at 110 °C for 5 hours using a hot air dryer to obtain a surface-treated glass powder. A resin mixture was prepared by mixing 50 parts by mass of Bis-GMA, 50 parts by mass of triethylene glycol dimethacrylate and 0.2 parts by mass of BPO. After kneading 50 parts by mass of the resin mixture and 50 parts by mass of the surface-treated glass powder until homogeneous, the kneaded material was heated at 100 °C for 4 hours in a nitrogen atmosphere to obtain a cured product. The obtained cured product was pulverized until the 50% particle diameter (D50) became 20 µm to obtain the organic-inorganic composite filler 3 (O3). The 50% particle diameter was measured by a laser diffraction/scattering type grain size distribution measuring apparatus (Microtrac MT3300EXII, manufactured by Microtrac Bell Co., Ltd.).

### [Manufacture of organic-inorganic composite filler 4 (O4)]

A surface treatment liquid (total mass: 11.5 parts by mass) was prepared by mixing 3.0 part by mass of 3-methacryloyloxypropyl trimethoxysilane, 0.5 part by mass of ion-exchanged water and 8.0 parts by mass of absolute ethanol. Next, a fluoroaluminosilicate glass was obtained in the same manner as in the manufacture of the acid-reactive glass powder 1 (G1). The obtained fluoroaluminosilicate glass was pulverized until the 50% particle diameter (D50) became 4 µm to obtain a fluoroaluminosilicate glass powder. This surface treatment liquid and 100 parts by mass of the above described fluoroaluminosilicate glass powder (50% particle diameter (D50): 4 µm) having the same components as the acid-reactive glass powder 1 (G1) were dry-mixed, and then heat-treated at 110 °C for 5 hours using a hot air dryer to obtain a surface-treated glass powder. A resin mixture was prepared by mixing 50 parts by mass of Bis-GMA, 50 parts by mass of triethylene glycol dimethacrylate and 0.2 parts by mass of BPO. After kneading 25 parts by mass of the resin mixture and 75 parts by mass of the surface-treated glass powder until homogeneous, the kneaded material was heated at 100 °C for 4 hours in a nitrogen atmosphere to obtain a cured product. The obtained cured product was pulverized until the 50% particle diameter (D50) became 55 µm to obtain the organic-inorganic composite filler 4 (O4). The 50% particle diameter was measured by a laser diffraction/scattering type grain size distribution measuring apparatus (Microtrac MT3300EXII, manufactured by Microtrac Bell Co., Ltd.).

### [Preparation of powder material and liquid material, or first paste and second paste]

The various components were mixed in the ratios shown in Tables 1 to 7 to prepare powder materials (Tables 1 to 4), liquid materials (Table 5), first pastes (Table 6) and a second pastes (Table 7).

### Composition of powder materials (% by mass)

**[Table 1]**

| | | **P1** | **P2** | **P3** | **P4** | **P5** | **P6** | **P7** |
|---|---|---|---|---|---|---|---|---|
| **(a) acid-reactive glass powder** | **G1** | | | | | | | |
| | **G2** | **89.2** | **89.2** | **89.2** | **89.2** | **80.9** | **95.8** | **97.2** |
| | **G3** | | | | | | | |
| | ***CA*** | | | | | | | |
| **(b) polvalkenoic acid** | **PCA1** | | | | | | | |
| **(d) polymeraizable monomer** | **14-AET** | | | | | | | |
| **(e) porous inorganic filler** | **PIF1** | **10.0** | | | | **19.0** | **4.0** | **2.0** |
| | **PIF2** | | **10.0** | | | | | |
| | **PIF3** | | | **10.0** | | | | |
| | **PIF4** | | | | **10.0** | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.02** | **0.02** | **0.02** | **0.02** | **0.01** | **0.02** | **0.02** |
| | **TSNa** | **0.77** | **0.77** | **0.77** | **0.77** | **0.08** | **0.17** | **0.77** |
| | **AA** | **0.01** | **0.01** | **0.01** | **0.01** | **0.01** | **0.01** | **0.01** |
| **(g) organic-inorganic composite filler** | **O1** | | | | | | | |
| | **02** | | | | | | | |
| | **03** | | | | | | | |
| | **04** | | | | | | | |
| **Others** | **Fuselex X** | | | | | | | |
| | **Aerosil R972** | | | | | | | |
| **Total (mass %)** | | **100.00** | **100.00** | **100.00** | **100.00** | **100.00** | **100.00** | **100.00** |

| | | **P8** | **P9** | **P10** | **P11** | **P12** | **P13** | **P14** |
|---|---|---|---|---|---|---|---|---|
| **(a) acid-reactive glass powder** | **G1** | | | | | | | |
| | **G2** | **84.2** | **84.2** | **84.2** | **84.2** | **89.1** | **97.2** | **89.2** |
| | **G3** | | | | | | | |
| | ***CA*** | | | | | | | |
| **(b) polvalkenoic acid** | **PCA1** | | | | | | | |
| **(d) polymeraizable monomer** | **14-AET** | | | | | | | |
| **(e) porous inorganic filler** | **PIF1** | **7.0** | **7.0** | **7.0** | **7.0** | **5.0** | **1.5** | **6.0** |
| | **PIF2** | | | | | | | |
| | **PIF3** | | | | | | | |
| | **PIF4** | | | | | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.02** | **0.02** | **0.02** | **0.02** | **0.07** | **0.02** | **0.02** |
| | **TSNa** | **0.77** | **0.77** | **0.77** | **0.77** | **0.76** | **0.77** | **0.77** |
| | **AA** | **0.01** | **0.01** | **0.01** | **0.01** | **0.07** | **0.01** | **0.01** |
| **(g) organic-inorganic composite filler** | **O1** | **8.0** | | | | **5.0** | **0.5** | **4.0** |
| | **02** | | **8.0** | | | | | |
| | **03** | | | **8.0** | | | | |
| | **04** | | | | **8.0** | | | |
| **Others** | **Fuselex X** | | | | | | | |
| | **Aerosil R972** | | | | | | | |
| **Total (mass %)** | | **100.00** | **100.00** | **100.00** | **100.00** | **100.00** | **100.00** | **100.00** |

### Composition of powder materials (% by mass)

**[Table 2]**

| | | **P15** | **P16** | **P17** | **P18** | **P19** | **P20** | **P21** |
|---|---|---|---|---|---|---|---|---|
| **(a) acid-reactive glass powder** | **G1** | | | | | | | |
| | **G2** | **94.6** | **80.7** | **73.7** | **71.4** | **73.2** | **95.2** | **96.0** |
| | **G3** | | | | | | | |
| | ***CA*** | | | | | | | |
| **(b) polvalkenoic acid** | **PCA1** | | | | | | | |
| **(d) polymeraizable monomer** | **4-AET** | | | | | | | |
| **(e) porous inorganic filler** | **PIF1** | **1.3** | **6.0** | **8.0** | **7.8** | **18.0** | **2.0** | **1.6** |
| | **PIF2** | | | | | | | |
| | **PIF3** | | | | | | | |
| | **PIF4** | | | | | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.02** | **0.02** | **0.02** | **0.02** | **0.02** | **0.02** | **0.02** |
| | **TSNa** | **0.77** | **0.77** | **0.77** | **0.77** | **0.77** | **0.77** | **0.77** |
| | **AA** | **0.01** | **0.01** | **0.01** | **0.01** | **0.01** | **0.01** | **0.01** |
| **(g) organic-inorganic composite filler** | **O1** | **3.3** | **12.5** | **17.5** | **20.0** | **8.0** | **2.0** | **1.6** |
| | **02** | | | | | | | |
| | **03** | | | | | | | |
| | **04** | | | | | | | |
| **Others** | **Fuselex X** | | | | | | | |
| | **Aerosil R972** | | | | | | | |
| **Total (mass %)** | | **100.00** | **100.00** | **100.00** | **100.00** | **I 100.00** | **100.00** | **100.00** |

| | | **P22** | **P23** | **P24** | **P25** | **P26** | **P27** | **P28** |
|---|---|---|---|---|---|---|---|---|
| **(a) acid-reactive glass powder** | **G1** | | | | | | | |
| | **G2** | **81.2** | **75.2** | **96.8** | **71.2** | **83.7** | **84.2** | **84.2** |
| | **G3** | | | | | | | |
| | **G4** | | | | | | | |
| **(b) polyalkenoic acid** | **PCA1** | | | | | | | |
| **(d) polymeraizable monomer** | **4-AET** | | | | | | | |
| **(e) porous inorganic filler** | **PIF1** | **9.0** | **12.0** | **1.4** | **14.0** | **8.5** | **10.0** | **5.0** |
| | **PIF2** | | | | | | | |
| | **PIF3** | | | | | | | |
| | **PIF4** | | | | | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.02** | **0.02** | **0.02** | **0.02** | **0.02** | **0.02** | **0.02** |
| | **TSNa** | **0.77** | **0.77** | **0.77** | **0.77** | **0.77** | **0.77** | **0.77** |
| | **AA** | **0.01** | **0.01** | **0.01** | **0.01** | **0.01** | **0.01** | **0.01** |
| **(g) organic-inorganic composite filler** | **O1** | **9.0** | **12.0** | **1.0** | **14.0** | **7.0** | **5.0** | **10.0** |
| | **02** | | | | | | | |
| | **03** | | | | | | | |
| | **04** | | | | | | | |
| **Others** | **Fuselex X** | | | | | | | |
| | **Aerosil R972** | | | | | | | |
| **Total (mass %)** | | **100.00** | **100.00** | **100.00** | **100.00** | **100.00** | **100.00** | **100.00** |

### Composition of powder materials (% by mass)

**[Table 3]**

| | | **P29** | **P30** | **P31** | **P32** | **P33** | **P34** | **P35** |
|---|---|---|---|---|---|---|---|---|
| **(a) acid-reactive glass powder** | **G1** | | | | | | **10.0** | |
| | **G2** | **81.7** | **82.2** | **84.2** | **70.0** | **90.2** | **74.2** | **67.1** |
| | **G3** | | | | | | | |
| | ***CA*** | | | | | | | **10.0** |
| **(b) polvalkenoic acid** | **PCA1** | | | | **10.0** | | | |
| **(d) polymeraizable monomer** | **4-AET** | | | | **1.0** | | | |
| **(e) porous inorganic filler** | **PIF1** | **5.2** | **12.0** | **4.0** | **8.0** | **4.0** | **7.0** | **10.0** |
| | **PIF2** | | | | | | | |
| | **PIF3** | | | | | | | |
| | **PIF4** | | | | | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.02** | **0.02** | **0.02** | **0.05** | **0.02** | **0.02** | **0.07** |
| | **TSNa** | **0.77** | **0.77** | **0.77** | **0.90** | **0.77** | **0.77** | **0.76** |
| | **AA** | **0.01** | **0.01** | **0.01** | **0.05** | **0.01** | **0.01** | **0.07** |
| **(g) organic-inorganic composite filler** | **O1** | **12.3** | **5.0** | **11.0** | **10.0** | **5.0** | **8.0** | **12.0** |
| | **02** | | | | | | | |
| | **03** | | | | | | | |
| | **04** | | | | | | | |
| **Others** | **Fuselex X** | | | | | | | |
| | **Aerosil R972** | | | | | | | |
| **Total (mass %)** | | **100.00** | **100.00** | **100.00** | **100.00** | **100.00** | **100.00** | **100.00** |

| | | **P36** | **P37** | **P38** | **P39** | **P40** | **P41** | **P42** |
|---|---|---|---|---|---|---|---|---|
| **(a) acid-reactive glass powder** | **G1** | | | | | | | |
| | **G2** | **71.1** | **99.2** | **98.2** | **79.2** | **84.2** | **84.2** | **89.2** |
| | **G3** | | | | | **15.0** | | |
| | **G4** | **10.0** | | | | | | |
| **(b) polyalkenoic acid** | **PCA1** | | | | | | | |
| **(d) polymeraizable monomer** | **4-AET** | | | | | | | |
| **(e) porous inorganic filler** | **PIF1** | **8.0** | | **1.0** | **20.0** | | | |
| | **PIF2** | | | | | | | |
| | **PIF3** | | | | | | | |
| | **PIF4** | | | | | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.07** | **0.02** | **0.02** | **0.02** | **0.02** | **0.02** | **0.02** |
| | **TSNa** | **0.76** | **0.77** | **0.77** | **0.77** | **0.77** | **0.77** | **0.77** |
| | **AA** | **0.07** | **0.01** | **0.01** | **0.01** | **0.01** | **0.01** | **0.01** |
| **(g) organic-inorganic composite filler** | **O1** | **10.0** | | | | | | **10.0** |
| | **02** | | | | | | | |
| | **03** | | | | | | | |
| | **04** | | | | | | | |
| **Others** | **Fuselex X** | | | | | | **15.0** | |
| | **Aerosil R972** | | | | | | | |
| **Total (mass %)** | | **100.00** | **100.00** | **100.00** | **100.00** | **100.00** | **100.00** | **100.00** |

### Composition of powder materials (% by mass)

**[Table 4]**

| | | **P43** | **P44** | **P45** | **P46** | **P47** | **P48** |
|---|---|---|---|---|---|---|---|
| **(a) acid-reactive glass powder** | **G1** | | | | | | |
| | **G2** | **87.2** | **89.2** | **89.2** | **89.2** | **89.2** | **96.6** |
| | **G3** | | | | | | |
| | **G4** | | | | | | |
| **(b) polvalkenoic acid** | **PCA1** | | | | | | |
| **(d) polymeraizable monomer** | **4-AET** | | | | | | |
| **(e) porous inorganic filler** | **PIF1** | | | | | | **1.4** |
| | **PIF2** | | | | | | |
| | **PIF3** | | | | | | |
| | **PIF4** | | | | | | |
| | **PIF5** | | **10.0** | | | | |
| | **PIF6** | | | **10.0** | | | |
| | **PIF7** | | | | **10.0** | | |
| | **PIF8** | | | | | **10.0** | |
| **(f) polymerization initiator** | **KPS** | **0.02** | **0.02** | **0.02** | **0.02** | **0.02** | **0.02** |
| | **TSNa** | **0.77** | **0.77** | **0.77** | **0.77** | **0.77** | **0.77** |
| | **AA** | **0.01** | **0.01** | **0.01** | **0.01** | **0.01** | **0.01** |
| **(g) organic-inorganic composite filler** | **O1** | **12.0** | | | | | **1.2** |
| | **02** | | | | | | |
| | **03** | | | | | | |
| | **04** | | | | | | |
| **Others** | **Fuselex X** | | | | | | |
| | **Aerosil R972** | | | | | | |
| **Total (mass %)** | | **100.00** | **100.00** | **100.00** | **100.00** | **100.00** | **100.00** |

### Composition of liquid materials (% by mass)

**[Table 5]**

| | | **L1** | **L2** | **L3** | **L4** | **L5** | **L6** | **L7** |
|---|---|---|---|---|---|---|---|---|
| **(b) polyalkenoic acid** | **PCA1** | **16.5** | **35.0** | **32.5** | **1.0** | **1.0** | **35.0** | |
| | **PCA2** | | | | | | | **16.5** |
| | **PCA3** | | | | | | | |
| **(c) water** | **IEW** | **22.2** | **55.0** | **52.5** | **1.0** | **13.0** | **30.0** | **22.2** |
| **(d) polymeraizable monomer** | **GDMA** | **20.0** | **2.5** | **3.0** | **30.0** | **20.0** | **10.0** | **20.0** |
| | **HEMA** | **20.0** | **5.0** | **6.0** | **30.0** | **40.0** | **15.0** | **20.0** |
| | **4-AET** | **10.0** | **1.0** | **2.5** | **20.0** | **12.0** | **5.0** | **10.0** |
| | **FAM** | **11.0** | **1.0** | **3.0** | **17.0** | **13.0** | **4.5** | **11.0** |
| | **Bis-GMA** | | | | | | | |
| **(f) polymerization initiator** | | **0.30** | **0.50** | **0.50** | **1.00** | **1.00** | **0.50** | **0.30** |
| **Others** | **Aerosil R972** | | | | | | | |
| **Total (mass %)** | | **100.00** | **100.00** | **100.00** | **100.00** | **100.00** | **100.00** | **100.00** |

| | | **L8** | **L9** | **L10** | **L11** | **L12** | **L13** | **L14** |
|---|---|---|---|---|---|---|---|---|
| **(b) polyalkenoic acid** | **PCA1** | **10.0** | **20.0** | **10.0** | | **10.0** | **2.5** | **13.6** |
| | **PCA2** | **6.5** | | | | | | |
| | **PCA3** | | | **6.5** | **16.5** | | | |
| **(c) water** | **IIEW** | **22.2** | **60.0** | **22.2** | **22.2** | **22.2** | **10.0** | **20.1** |
| **(d) polymeraizable monomer** | **GDMA** | **20.0** | **7.5** | **20.0** | **20.0** | **23.0** | **32.0** | **22.5** |
| | **HEMA** | **20.0** | **10.0** | **20.0** | **20.0** | **23.0** | **32.0** | **22.5** |
| | **4-AET** | **10.0** | **1.0** | **10.0** | **10.0** | **10.5** | **10.7** | **10.0** |
| | **FAM** | **11.0** | **1.0** | **11.0** | **11.0** | **11.0** | **12.5** | **11.0** |
| | **Bis-GMA** | | | | | | | |
| **(f) polymerization initiator** | **CQ** | **0.30** | **0.50** | **0.30** | **0.30** | **0.30** | **0.30** | **0.30** |
| **Others** | **Aerosil R972** | | | | | | | |
| **Total (mass %)** | | **100.00** | **100.00** | **100.00** | **100.00** | **100.00** | **100.00** | **100.00** |

### Composition of first pastes (% by mass)

**[Table 6]**

| | | **AP1** | **AP2** | **AP3** | **AP4** | **AP5** |
|---|---|---|---|---|---|---|
| **(a) acid-reactive glass powder** | **G1** | | | | | |
| | **G2** | **57.0** | **50.0** | **71.0** | **64.0** | **56.0** |
| | **G3** | | | | | |
| | **G4** | | | | | |
| **(d) polymeraizable monomer** | **GDMA** | **11.5** | **14.0** | **11.5** | **8.0** | **11.5** |
| | **HEMA** | **11.5** | **14.0** | **11.5** | **8.0** | **11.5** |
| | **4-AET** | | | | | |
| | **Bis-GMA** | **1.5** | **2.0** | **1.0** | **3.0** | **1.0** |
| **(e) porous inorganic filler** | **PIF1** | **15.0** | | | | |
| | **PIF2** | | **6.0** | | **5.0** | |
| | **PIF3** | | | | | |
| | **PIF4** | | | | | |
| | **PIF5** | | | | | |
| | **PIF6** | | | | | |
| | **PIF7** | | | | | |
| | **PIF8** | | | | | |
| **(f) polymerization initiator** | **KPS** | **0.02** | **0.02** | **0.02** | **0.02** | **0.02** |
| | **TSNa** | **0.77** | **0.77** | **0.76** | **0.77** | **0.76** |
| | **AA** | **0.01** | **0.01** | **0.02** | **0.01** | **0.02** |
| **(g) organic-inorganic composite filler** | **O1** | | **9.0** | | **9.0** | **15.0** |
| | **02** | | | | | |
| | **03** | | | | | |
| | **04** | | | | | |
| **Others** | **Fuselex X** | | | | | |
| | **Aerosil R972** | **2.7** | **4.2** | **4.2** | **2.2** | **4.2** |
| **Total (mass %)** | | **100.00** | **100.00** | **100.00** | **100.00** | **100.00** |

### Composition of second pastes (% by mass)

**[Table 7]**

| | | **BP1** | **BP2** | **BP3** |
|---|---|---|---|---|
| **(b) polyalkenoic acid** | **PCA1** | **15.0** | **25.0** | **15.0** |
| | **PCA2** | | | |
| | **PCA3** | | | |
| **(c) water** | **IEW** | **22.0** | **32.0** | **19.0** |
| **(d) polymeraizable monomer** | **GDMA** | **20.0** | **10.0** | **21.0** |
| | **HEMA** | **20.0** | **10.0** | **21.0** |
| | **4-AET** | **9.5** | **10.0** | **10.0** |
| | **FAM** | **10.5** | **10.0** | **10.0** |
| | **Bis-GMA** | **2.0** | **2.0** | **3.0** |
| **(f) polymerization initiator** | **CQ** | **0.30** | **0.30** | **0.30** |
| **Others** | **Aerosil R972** | **0.7** | **0.7** | **0.7** |
| **Total (mass %)** | | **100.00** | **100.00** | **100.00** |

### [Dental resin-reinforced glass ionomer cement compositions]

Dental resin-reinforced glass ionomer cement compositions for filling or luting in which the powder material and the liquid material, or the first paste and the second paste were combined in the powder-liquid ratio or paste ratio (mass ratio) shown in Tables 8 to 12 respectively (Examples 1 to 63 and Comparative Examples 1 to 9) were evaluated for mixability, stringiness of mixed material, shaping start time, compressive strength and contrast ratio. For Examples 1 to 5, 8 to 10, 12 to 33, 36, 40, 43, 44, 46, 48, 50, 53, 55 to 63 and Comparative Examples 1, 4 to 6, and 9, which are the compositions for filling, all of the above items were evaluated, and for Examples 6, 7, 11, 34, 35, 37 to 39, 41, 42, 45, 47, 49, 51, 52 and Comparative Examples 2, 3, 7, and 8 which are the compositions for luting, items other than the shaping start time were evaluated. The evaluation method is as follows.

### [Mixability]

Under an environment of a temperature of 23 ± 1 °C and a humidity 50 ± 10 %, the powder material and the liquid material, or the first paste and the second paste, of the dental resin-reinforced glass ionomer cement compositions of the Examples or Comparative Examples were mixed using a plastic spatula in the ratios shown in Tables 8 to 12. The total amount of the powder material and the liquid material, or the first paste and the second paste, was 360 mg. At this time, the time from the start of mixing was used as the base point, and the time until the mixed material became homogeneous was measured. Three evaluators each performed three times of evaluation procedures, and the most common evaluation result was taken as the evaluation result of the measurement object. When the evaluation result was A, B or C, it was determined to have good mixability.

### -Evaluation criteria-

### [Powder-liquid type]

A: Time required for the mixed material to become uniform was less than 40 seconds.
B: Time required for the mixed material to become uniform was 40 seconds or more and less than 50 seconds.
C: Time required for the mixed material to become uniform was 50 seconds or more and less than 55 seconds.
D: Time required for the mixed material to become uniform was 55 seconds or longer or the mixed material did not become uniform.

### [Paste type]

A: Time required for the mixed material to become uniform was less than 10 seconds.
B: Time required for the mixed material to become uniform was 10 seconds or more and less than 15 seconds.
C: Time required for the mixed material to become uniform was 15 seconds or more and less than 20 seconds.
D: Time required for the mixed material to become uniform was 20 seconds or longer.

### [Stringiness of mixed material]

Under an environment of a temperature of 23 ± 1 °C and a humidity 50 ± 10 %, the powder material and the liquid material, or the first paste and the second paste, of the dental resin-reinforced glass ionomer cement compositions of the Examples or Comparative Examples were mixed using a plastic spatula in the ratios shown in Tables 8 to 12. The total amount of the powder material and the liquid material, or the first paste and the second paste, was 360 mg. Immediately after the end of mixing, the mixed material was filled into a plastic simulated cavity (having 4 mm x 8 mm x 2 mm and simulating a class I cavity), and the excess was scraped off to make the surface flat. Ten seconds after the end of mixing, the cylindrical tip (diameter φ1.5 mm) of a metallic instrument (MiCD instrument manufactured by SHOFU INC.) was vertically submerged 0.5 mm into the mixed material, and the instrument was immediately and gently pulled up. At this time, the degree of stringiness of the mixed material was evaluated according to the following evaluation criteria. Three evaluators each performed three evaluations, and the most common evaluation result was taken as the evaluation result of the measurement object. When the evaluation result was A or B, it was determined to have good mixed material property with little stringiness.

### -Evaluation criteria-

A: No stringiness.
B: Slightly stringiness.
C: Significant stringiness.

### [Shaping start time]

Under an environment of a temperature of 23 ± 1 °C and a humidity 50 ± 10 %, the powder material and the liquid material, or the first paste and the second paste, of the dental resin-reinforced glass ionomer cement compositions of the Examples or Comparative Examples were mixed using a plastic spatula in the ratios shown in Tables 8 to 12. The total amount of the powder material and the liquid material, or the first paste and the second paste, was 360 mg. Immediately after the end of mixing, the mixed material was filled into a plastic simulated cavity (having 4 mm x 8 mm x 2 mm and simulating a class I cavity), and the excess was scraped off to make the surface flat. After 20 second from the end of mixing, at 10-second intervals, the cylindrical tip (diameter φ1.5 mm) of a metal instrument (MiCD instrument manufactured by SHOFU INC.) was vertically submerged 0.5 mm into the mixed material, and the instrument was immediately and gently pulled up. At this time, the degree of stringiness of the mixed material was evaluated. The time from the end of mixing was used as the base point, and the time until the stringiness of the mixed material was reduced and it was possible to perform shaping operation was measured. Three evaluators each performed three evaluations, and the most common evaluation result was taken as the evaluation result of the measurement object. When the evaluation result was A, B or C, it was determined that the shaping start time was early.

### -Evaluation criteria-

A: 20 seconds after the end of mixing, the stringiness of the mixed material was reduced and shaping operations were possible.
B: 30 seconds after the end of mixing, the stringiness of the mixed material was reduced and shaping operations were possible.
C: 40 seconds after the end of mixing, the stringiness of the mixed material was reduced and shaping operations were possible.
D: After 50 seconds or more from the end of mixing, the stringiness of the mixed material is reduced and shaping operation was possible.

### [Compressive strength]

Under an environment of a temperature of 23 ± 1 °C and a humidity 50 ± 10 %, the powder material and the liquid material, or the first paste and the second paste, of the dental resin-reinforced glass ionomer cement compositions of the Examples or Comparative Examples were mixed using a plastic spatula in the ratios shown in Tables 8 to 12. The mixed material was filled into a stainless mold (cylindrical shape having an inner diameter of 4 mm and a height of 6 mm) and irradiated with light for 10 seconds using a dental polymerization LED light irradiator (PEN Bright, manufactured by SHOFU INC.), and then allowed to stand in a thermo-hygrostat chamber at a temperature of 37 °C and a humidity of 90% or more. After standing for 1 hour, the set product was removed from the mold and used as a test specimen. After immersing the test specimen in ion-exchanged water at 37 °C for 24 hours from the end of mixing, the compressive strength of the test specimen was measured at a crosshead speed of 1 mm/min using an Instron universal testing machine (model: 5567A) in accordance with ISO 9917-1:2007. Measurement was performed five times, and the average of the measurement results was evaluated based on the following evaluation criteria to determine the evaluation result of the measurement object. When the evaluation result was A, B or C, it was determined to have good mechanical characteristic.

### -Evaluation criteria-

### [Composition for filling]

A: Compressive strength was 180 MPa or more.
B: Compressive strength was 160 MPa or more and less than 180 MPa.
C: Compressive strength was 150 MPa or more and less than 160 MPa.
D: Compressive strength was less than 150 MPa.

### [Compositions for luting]

A: Compressive strength was 100 MPa or more
B: Compressive strength was 80 MPa or more and less than 100 MPa.
C: Compressive strength was 70 MPa or more and less than 80 MPa.
D: Compressive strength was less than 70 MPa.

### [Contrast ratio]

Under an environment of a temperature of 23 ± 1 °C and a humidity 50 ± 10 %, the powder material and the liquid material, or the first paste and the second paste, of the dental resin-reinforced glass ionomer cement compositions of the Examples or Comparative Examples were mixed using a plastic spatula in the ratios shown in Tables 8 to 12. The mixed material was filled into a stainless ring shape mold (having an inner diameter of 12 mm and a height of 1 mm), irradiated with light for 10 seconds using a dental polymerization LED light irradiator (PEN Bright, manufactured by SHOFU INC.), and then allowed to stand in a thermo-hygrostat chamber at a temperature of 37 °C and a humidity of 90% or more. After standing for 1 hour, the set product was removed from the mold and used as a test specimen. After immersing the test specimen in ion-exchanged water at 37 °C for 24 hours from the end of mixing, the Y value (Yw) on the white plate and the Y value (Yb) on the black plate for the test specimen were measured using a colorimeter (spectra photometer: CM-3500d), and the contrast ratio was calculated by finding the ratio (Yb/Yw) of these values. Measuremnt was performed three times, and the average of the measurement results was evaluated based on the following evaluation criteria to determine the evaluation result of the measurement object. When the evaluation result was A, B or C, it was determined to have good transparency.

### -Evaluation criteria-

A: Contrast ratio was less than 0.68.
B: Contrast ratio was 0.68 or more and less than 0.70.
C: Contrast ratio was 0.70 or more and less than 0.71.
D: Contrast ratio was more than 0.71.

The results of the evaluation of each composition in the examples and comparative examples according to the above test method are shown in Tables 8 to 12.

### Evaluation results of Examples 1 to 15

**[Table 8]**

| | | **Example 1** | **Example 2** | **Example 3** | **Example 4** | **Example 5** | **Example 6** | **Example 7** | **Example 8** |
|---|---|---|---|---|---|---|---|---|---|
| **Type** | | **Powder Liquid** | **Powder Liquid** | **Powder Liquid** | **Powder Liquid** | **Powder Liquid** | **Powder Liquid** | **Powder Liquid** | **Powder Liquid** |
| **Powder or First paste** | | **P1** | **P2** | **P3** | **P4** | **P5** | **P5** | **P5** | **P6** |
| **Liquid or Second paste** | | **L1** | **L1** | **L1** | **L1** | **L1** | **L1** | **L2** | **L3** |
| **Powder-Liquid ratio (mass ratio) or Paste ratio (mass ratio)** | | **3.6/1.0** | **3.6/1.0** | **3.6/1.0** | **3.6/1.0** | **3.6/1.0** | **2.3/1.0** | **1.0/1.0** | **4.5/1.0** |
| **Use** | | **Filling** | **Filling** | **Filling** | **Filling** | **Filling** | **Luting** | **Luting** | **Filling** |
| **(a) acid-reactive glass powder** | **G1** | | | | | | | | |
| | **G2** | **69.9** | **69.9** | **69.9** | **69.9** | **63.35** | **56.4** | **40.4** | **78.4** |
| | **G3** | | | | | | | | |
| | **G4** | | | | | | | | |
| **(b) polyalkenoic acid** | **PCA1** | **3.6** | **3.6** | **3.6** | **3.6** | **3.6** | **5.0** | **17.5** | **5.9** |
| | **PCA2** | | | | | | | | |
| | **PCA3** | | | | | | | | |
| **(c) water** | **IEW** | **4.8** | **4.8** | **4.8** | **4.8** | **4.8** | **6.7** | **27.5** | **9.5** |
| **(d) polymeraizable monomer** | **GDMA** | **4.3** | **4.3** | **4.3** | **4.3** | **4.3** | **6.1** | **1.3** | **0.5** |
| | **HEMA** | **4.3** | **4.3** | **4.3** | **4.3** | **4.3** | **6.1** | **2.5** | **1.1** |
| | **4-AET** | **2.2** | **2.2** | **2.2** | **2.2** | **2.2** | **3.0** | **0.5** | **0.5** |
| | **FAM** | **2.4** | **2.4** | **2.4** | **2.4** | **2.4** | **3.3** | **0.5** | **0.5** |
| | **Bis-GMA** | | | | | | | | |
| **(e) porous inorganic filler** | **PIF1** | **7.8** | | | | **14.9** | **13.2** | **9.5** | **3.3** |
| | **PIF2** | | **7.8** | | | | | | |
| | **PIF3** | | | **7.8** | | | | | |
| | **PIF4** | | | | **7.8** | | | | |
| | **PIF5** | | | | | | | | |
| | **PIF6** | | | | | | | | |
| | **PIF7** | | | | | | | | |
| | **PIF8** | | | | | | | | |
| **(f) polymerization initiator** | **KPS** | **0.02** | **0.02** | **0.02** | **0.02** | **0.01** | **0.01** | **0.01** | **0.02** |
| | **TSNa** | **0.60** | **0.60** | **0.60** | **0.60** | **0.06** | **0.09** | **0.04** | **0.18** |
| | **AA** | **0.01** | **0.01** | **0.01** | **0.01** | **0.01** | **0.01** | **0.01** | **0.01** |
| | **CQ** | **0.07** | **0.07** | **0.07** | **0.07** | **0.07** | **0.09** | **0.24** | **0.09** |
| **(g) organic-inorganic composite filler** | **O1** | | | | | | | | |
| | **O2** | | | | | | | | |
| | **O3** | | | | | | | | |
| | **O4** | | | | | | | | |
| **Others** | **Fuselex X** | | | | | | | | |
| | **Aerosil R972** | | | | | | | | |
| **Total (mass %)** | | **100.00** | **100.00** | **100.00** | **100.00** | **100.00** | **100.00** | **100.00** | **100.00** |
| **Total content \|(e) + (g)\| (mass%)** | | **7.8** | **7.8** | **7.8** | **7.8** | **14.9** | **13.2** | **9.5** | **3.3** |
| **Mass ratio \|(g)/(e)\|** | | **-** | **-** | **-** | **-** | **-** | **-** | **-** | **-** |
| **Mixability** | | **C** | **C** | **C** | **C** | **C** | **C** | **C** | **C** |
| **Stringiness of mixed material** | | **A** | **A** | **A** | **A** | **A** | **A** | **A** | **A** |
| **Shaping start time (see)** | | **20** | **20** | **20** | **20** | **20** | **-** | **-** | **20** |
| **Evaluation** | | **A** | **A** | **A** | **A** | **A** | **-** | **-** | **A** |
| **Compressive strength (Mpa)** | | **218** | **214** | **210** | **208** | **175** | **97** | **85** | **189** |
| **Evaluation** | | **A** | **A** | **A** | **A** | **B** | **B** | **B** | **A** |
| **Contrast ratio** | | **0.63** | **0.64** | **0.64** | **0.63** | **0.64** | **0.64** | **0.59** | **0.65** |
| **Evaluation** | | **A** | **A** | **A** | **A** | **A** | **A** | **A** | **A** |

| | | **Example 9** | **Example 10** | **Example 11** | **Example 12** | **Example 13** | **Example 14** | **Example 15** |
|---|---|---|---|---|---|---|---|---|
| **Type** | | **Powder Liquid** | **Powder Liquid** | **Powder Liquid** | **Powder Liquid** | **Powder Liquid** | **Powder Liquid** | **Powder Liquid** |
| **Powder or First paste** | | **Total (mass %** | **0** | **100** | **100** | **100** | **100** | **100** |
| **Liquid or Second paste** | | **L4** | **L1** | **L1** | **L3** | **L1** | **L2** | **L4** |
| **Powder-Liquid ratio (mass ratio) or Paste ratio (mass ratio)** | | **4.8/1.0** | **3.6/1.0** | **2.0/1.0** | **3.6/1.0** | **3.6/1.0** | **4.5/1.0** | **4.8/1.0** |
| **Use** | | **Filling** | **Filling** | **Luting** | **Filling** | **Filling** | **Filling** | **Filling** |
| **(a) acid-reactive glass powder** | **G1** | | | | | | | |
| | **G2** | **80.4** | **65.9** | **56.1** | **65.8** | **65.9** | **72.8** | **80.5** |
| | **G3** | | | | | | | |
| | **G4** | | | | | | | |
| **(b) polyalkenoic acid** | **PCA1** | **0.2** | **3.6** | **5.5** | **7.1** | **3.6** | **6.4** | **0.2** |
| | **PCA2** | | | | | | | |
| | **PCA3** | | | | | | | |
| **(c) water** | **IEW** | **0.2** | **4.8** | **7.4** | **11.4** | **4.8** | **10.0** | **0.2** |
| **(d) polymeraizable monomer** | **GDMA** | **5.2** | **4.3** | **6.7** | **0.7** | **4.3** | **0.5** | **5.2** |
| | **HEMA** | **5.2** | **4.3** | **6.7** | **1.3** | **4.3** | **0.9** | **5.2** |
| | **4-AET** | **3.4** | **2.2** | **3.3** | **0.5** | **2.2** | **0.2** | **3.4** |
| | **FAM** | **2.9** | **2.4** | **3.7** | **0.7** | **2.4** | **0.2** | **2.9** |
| | **Bis-GMA** | | | | | | | |
| **(e) porous inorganic filler** | **PIF1** | **1.7** | **5.5** | **4.7** | **5.5** | **5.5** | **4.1** | **1.2** |
| | **PIF2** | | | | | | | |
| | **PIF3** | | | | | | | |
| | **PIF4** | | | | | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.02** | **0.02** | **0.01** | **0.02** | **0.02** | **0.06** | **0.02** |
| | **TSNa** | **0.60** | **0.60** | **0.48** | **0.56** | **0.60** | **0.59** | **0.60** |
| | **AA** | **0.01** | **0.01** | **0.01** | **0.01** | **0.01** | **0.06** | **0.01** |
| | **CQ** | **0.17** | **0.07** | **0.10** | **0.11** | **0.07** | **0.09** | **0.17** |
| **(g) organic-inorganic composite filler** | **O1** | | **6.3** | | | | **4.1** | **0.4** |
| | **O2** | | | **5.3** | | | | |
| | **O3** | | | | **6.3** | | | |
| | **O4** | | | | | **6.3** | | |
| **Others** | **Fuselex X** | | | | | | | |
| | **Aerosil R972** | | | | | | | |
| **Total (mass %)** | | **100.00** | **100.00** | **100.00** | **100.00** | **100.00** | **100.00** | **100.00** |
| **Total content \|(e) + (g)\| (mass%)** | | **1.7** | **11.8** | **10.0** | **11.8** | **11.8** | **8.2** | **1.6** |
| **Mass ratio \|(g)/(e)\|** | | **-** | **1.15** | **1.13** | **1.15** | **1.15** | **1.00** | **0.33** |
| **Mixability** | | **C** | **A** | **A** | **A** | **A** | **A** | **C** |
| **Stringiness of mixed material** | | **B** | **A** | **A** | **A** | **A** | **A** | **B** |
| **Shaping start time (sec)** | | **40** | **20** | - | **20** | **20** | **20** | **40** |
| **Evaluation** | | **C** | **A** | - | **A** | **A** | **A** | **C** |
| **Compressive strength (Mpa)** | | **164** | **216** | **122** | **185** | **198** | **162** | **162** |
| **Evaluation** | | **B** | **A** | **A** | **A** | **A** | **B** | **B** |
| **Contrast ratio** | | **0.62** | **0.65** | **0.64** | **0.65** | **0.65** | **0.63** | **0.63** |
| **Evaluation** | | **A** | **A** | **A** | **A** | **A** | **A** | **A** |

### Evaluation results of Examples 16 to 30

**[Table 9]**

| | | **Example 16** | **Example 17** | **Example 18** | **Example 19** | **Example 20** | **Example 21** | **Example 22** | **Example 23** |
|---|---|---|---|---|---|---|---|---|---|
| **Type** | | **Powder Liquid** | **Powder Liquid** | **Powder Liquid** | **Powder Liquid** | **Powder Liquid** | **Powder Liquid** | **Powder Liquid** | **Powder Liquid** |
| **Powder or First paste** | | **0** | **P15** | **P16** | **P17** | **P18** | **P19** | **P20** | **P21** |
| **Liquid or Second paste** | | **L1** | **L1** | **L1** | **L1** | **L1** | **L1** | **L1** | **L1** |
| **Powder-Liquid ratio (mass ratio) or Paste ratio (mass ratio)** | | **3.6/1.0** | **3.6/1.0** | **3.6/1.0** | **3.6/1.0** | **3.6/1.0** | **3.6/1.0** | **3.6/1.0** | **3.6/1.0** |
| **Use** | | **Filling** | **Filling** | **Filling** | **Filling** | **Filling** | **Filling** | **Filling** | **Filling** |
| **(a) acid-reactive glass powder** | **G1** | | | | | | | | |
| | **G2** | **69.9** | **74.1** | **63.2** | **57.7** | **55.9** | **57.3** | **74.5** | **75.1** |
| | **G3** | | | | | | | | |
| | **G4** | | | | | | | | |
| **(b) polyalkenoic acid** | **PCA1** | **3.6** | **3.6** | **3.6** | **3.6** | **3.6** | **3.6** | **3.6** | **3.6** |
| | **PCA2** | | | | | | | | |
| | **PCA3** | | | | | | | | |
| **(c) water** | **IEW** | **4.8** | **4.8** | **4.8** | **4.8** | **4.8** | **4.8** | **4.8** | **4.8** |
| **(d) polymeraizable monomer** | **GDMA** | **4.3** | **4.3** | **4.3** | **4.3** | **4.3** | **4.3** | **4.3** | **4.3** |
| | **HEMA** | **4.3** | **4.3** | **4.3** | **4.3** | **4.3** | **4.3** | **4.3** | **4.3** |
| | **4-AET** | **2.2** | **2.2** | **2.2** | **2.2** | **2.2** | **2.2** | **2.2** | **2.2** |
| | **FAM** | **2.4** | **2.4** | **2.4** | **2.4** | **2.4** | **2.4** | **2.4** | **2.4** |
| | **Bis-GMA** | | | | | | | | |
| **(e) porous inorganic filler** | **PIF1** | **4.7** | **1.0** | **4.7** | **6.3** | **6.1** | **14.1** | **1.6** | **1.3** |
| | **PIF2** | | | | | | | | |
| | **PIF3** | | | | | | | | |
| | **PIF4** | | | | | | | | |
| | **PIF5** | | | | | | | | |
| | **PIF6** | | | | | | | | |
| | **PIF7** | | | | | | | | |
| | **PIF8** | | | | | | | | |
| **(f) polimerization initiator** | **KPS** | **0.02** | **0.02** | **0.02** | **0.02** | **0.02** | **0.02** | **0.02** | **0.02** |
| | **TSNa** | **0.60** | **0.60** | **0.60** | **0.60** | **0.60** | **0.60** | **0.60** | **0.60** |
| | **AA** | **0.01** | **0.01** | **0.01** | **0.01** | **0.01** | **0.01** | **0.01** | **0.01** |
| | **CQ** | **0.07** | **0.07** | **0.07** | **0.07** | **0.07** | **0.07** | **0.07** | **0.07** |
| **(g) organic-inorganic composite filler** | **O1** | **3.1** | **2.6** | **9.8** | **13.7** | **15.7** | **6.3** | **1.6** | **1.3** |
| | **O2** | | | | | | | | |
| | **O3** | | | | | | | | |
| | **O4** | | | | | | | | |
| **Others** | **Fuselex X** | | | | | | | | |
| | **Aerosil R972** | | | | | | | | |
| **Total (mass %)** | | **100.00** | **100.00** | **100.00** | **100.00** | **100.00** | **100.00** | **100.00** | **100.00** |
| **Total content \|(e) +(g)\| (mass%)** | | **7.8** | **3.6** | **14.5** | **20.0** | **21.8** | **20.4** | **3.2** | **2.6** |
| **Mass ratio \|(g)/(e)\|** | | **0.66** | **2.60** | **2.09** | **2.17** | **2.57** | **0.45** | **1.00** | **1.00** |
| **Mixability** | | **B** | **B** | **B** | **B** | **B** | **C** | **A** | **A** |
| **Stringiness of mixed material** | | **A** | **A** | **A** | **A** | **A** | **A** | **A** | **B** |
| **Shaping start time (sec)** | | **20** | **20** | **20** | **20** | **20** | **20** | **20** | **30** |
| **Evaluation** | | **A** | **A** | **A** | **A** | **A** | **A** | **A** | **B** |
| **Compressive strength (Mpa)** | | **220** | **218** | **210** | **167** | **159** | **158** | **212** | **220** |
| **Evaluation** | | **A** | **A** | **A** | **B** | **C** | **C** | **A** | **A** |
| **Contrast ratio** | | **0.64** | **0.70** | **0.67** | **0.69** | **0.70** | **0.65** | **0.64** | **0.63** |
| **Evaluation** | | **A** | **C** | **A** | **B** | **C** | **A** | **A** | **A** |

| | | **Example 24** | **Example 25** | **Example 26** | **Example 27** | **Example 28** | **Example 29** | **Example 30** |
|---|---|---|---|---|---|---|---|---|
| **Type** | | **Powder Liquid** | **Powder Liquid** | **Powder Liquid** | **Powder Liquid** | **Powder Liquid** | **Powder Liquid** | **Powder Liquid** |
| **Powder or First paste** | | **Aerosil R972** | **0** | **0** | **0** | **0** | **0** | **0** |
| **Liquid or Second paste** | | **L1** | **L1** | **L1** | **L1** | **L1** | **L1** | **L1** |
| **Powder-Liquid ratio (mass ratio) or Paste ratio (mass ratio)** | | **3.6/1.0** | **3.6/1.0** | **3.6/1.0** | **3.6/1.0** | **3.6/1.0** | **3.6/1.0** | **3.6/1.0** |
| **Use** | | **Filling** | **Filling** | **Filling** | **Filling** | **Filling** | **Filling** | **Filling** |
| **(a) acid-reactive glass powder** | **G1** | | | | | | | |
| | **G2** | **63.7** | **58.9** | **75.8** | **55.7** | **65.5** | **66.0** | **66.0** |
| | **G3** | | | | | | | |
| | **G4** | | | | | | | |
| **(b) polyalkenoic acid** | **PCA1** | **3.6** | **3.6** | **3.6** | **3.6** | **3.6** | **3.6** | **3.6** |
| | **PCA2** | | | | | | | |
| | **PCA3** | | | | | | | |
| **(c) water** | **IEW** | **4.8** | **4.8** | **4.8** | **4.8** | **4.8** | **4.8** | **4.8** |
| **(d) polymeraizable monomer** | **GDMA** | **4.3** | **4.3** | **4.3** | **4.3** | **4.3** | **4.3** | **4.3** |
| | **HEMA** | **4.3** | **4.3** | **4.3** | **4.3** | **4.3** | **4.3** | **4.3** |
| | **4-AET** | **2.2** | **2.2** | **2.2** | **2.2** | **2.2** | **2.2** | **2.2** |
| | **FAM** | **2.4** | **2.4** | **2.4** | **2.4** | **2.4** | **2.4** | **2.4** |
| | **Bis-GMA** | | | | | | | |
| **(e) porous inorganic filler** | **PIF1** | **7.0** | **9.4** | **1.1** | **11.0** | **6.7** | **7.8** | **3.9** |
| | **PIF2** | | | | | | | |
| | **PIF3** | | | | | | | |
| | **PIF4** | | | | | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.02** | **0.02** | **0.02** | **0.02** | **0.02** | **0.02** | **0.02** |
| | **TSNa** | **0.60** | **0.60** | **0.60** | **0.60** | **0.60** | **0.60** | **0.60** |
| | **AA** | **0.01** | **0.01** | **0.01** | **0.01** | **0.01** | **0.01** | **0.01** |
| | **CQ** | **0.07** | **0.07** | **0.07** | **0.07** | **0.07** | **0.07** | **0.07** |
| **(g) organic-inorganic composite filler** | **O1** | **7.0** | **9.4** | **0.8** | **11.0** | **5.5** | **3.9** | **7.8** |
| | **O2** | | | | | | | |
| | **O3** | | | | | | | |
| | **O4** | | | | | | | |
| **Others** | **Fuselex X** | | | | | | | |
| | **Aerosil R972** | | | | | | | |
| **Total (mass %)** | | **100.00** | **100.00** | **100.00** | **100.00** | **100.00** | **100.00** | **100.00** |
| **Total content \|(e)+(e)\| (mass%)** | | **14.0** | **18.8** | **1.9** | **22.0** | **12.2** | **11.7** | **11.7** |
| **Mass ratio \|(g)/(e)\|** | | **1.00** | **1.00** | **0.73** | **1.00** | **0.82** | **0.50** | **2.00** |
| **Mixability** | | **A** | **B** | **A** | **B** | **A** | **B** | **A** |
| **Stringiness of mixed material** | | **A** | **A** | **B** | **A** | **A** | **A** | **A** |
| **Shaping start time (sec)** | | **20** | **20** | **40** | **20** | **20** | **20** | **20** |
| **Evaluation** | | **A** | **A** | **C** | **A** | **A** | **A** | **A** |
| **Compressive strength (Mpa)** | | **210** | **188** | **219** | **158** | **203** | **210** | **201** |
| **Evaluation** | | **A** | **A** | **A** | **C** | **A** | **A** | **A** |
| **Contrast ratio** | | **0.65** | **0.68** | **0.64** | **0.68** | **0.64** | **0.63** | **0.66** |
| **Evaluation** | | **A** | **B** | **A** | **B** | **A** | **A** | **A** |

### Evaluation results of Examples 31 to 45

**[Table 10]**

| | | **Example 31** | **Example 32** | **Example 33** | **Example 34** | **Example 35** | **Example 36** | **Example 37** | **Example 38** |
|---|---|---|---|---|---|---|---|---|---|
| **Type** | | **Powder Liquid** | **Powder Liquid** | **Powder Liquid** | **Paste** | **Paste** | **Paste** | **Powder Liquid** | **Powder Liquid** |
| **Powder or First paste** | | **P29** | **P30** | **P31** | **AP1** | **AP2** | **AP4** | **P32** | **P33** |
| **Liquid or Second paste** | | **L1** | **L1** | **L1** | **BP1** | **BP2** | **BP3** | **L4** | **L3** |
| **Powder-Liquid ratio (mass ratio) or Paste ratio (mass ratio)** | | **3.6/1.0** | **3.6/1.0** | **3.6/1.0** | **1.5/1.0** | **1.5/1.0** | **1.6/1.0** | **1.0/1.0** | **1.0/1.0** |
| **Use** | | **Filling** | **Filling** | **Filling** | **Luting** | **Luting** | **Filling** | **Luting** | **Luting** |
| **(a) acid-reactive glass powder** | **G1** | | | | | | | | |
| | **G2** | **64.0** | **64.4** | **66.0** | **34.2** | **30.0** | **39.4** | **35.0** | **45.1** |
| | **G3** | | | | | | | | |
| | **G4** | | | | | | | | |
| **(b) polyalkenoic acid** | **PCA1** | **3.6** | **3.6** | **3.6** | **6.0** | **10.0** | **5.8** | **5.5** | **16.3** |
| | **PCA2** | | | | | | | | |
| | **PCA3** | | | | | | | | |
| **(c) water** | **IEW** | **4.8** | **4.8** | **4.8** | **8.8** | **12.8** | **7.3** | **0.5** | **26.2** |
| **(d) polymeraizable monomer** | **GDMA** | **4.3** | **4.3** | **4.3** | **14.9** | **12.4** | **13.0** | **15.0** | **1.5** |
| | **HEMA** | **4.3** | **4.3** | **4.3** | **14.9** | **12.4** | **13.0** | **15.0** | **3.0** |
| | **4-AET** | **2.2** | **2.2** | **2.2** | **3.8** | **4.0** | **3.8** | **10.5** | **1.3** |
| | **FAM** | **2.4** | **2.4** | **2.4** | **4.2** | **4.0** | **3.8** | **8.5** | **1.5** |
| | **Bis-GMA** | | | | **1.7** | **2.0** | **3.0** | | |
| **(e) porous inorganic filler** | **PIF1** | **4.1** | **9.4** | **3.1** | **9.0** | **3.6** | | **4.0** | **2.0** |
| | **PIF2** | | | | | | **3.1** | | |
| | **PIF3** | | | | | | | | |
| | **PIF4** | | | | | | | | |
| | **PIF5** | | | | | | | | |
| | **PIF6** | | | | | | | | |
| | **PIF7** | | | | | | | | |
| | **PIF8** | | | | | | | | |
| **(f) polimerization initiator** | **KPS** | **0.02** | **0.02** | **0.02** | **0.01** | **0.01** | **0.01** | **0.03** | **0.01** |
| | **TSNa** | **0.60** | **0.60** | **0.60** | **0.46** | **0.46** | **0.56** | **0.44** | **0.33** |
| | **AA** | **0.01** | **0.01** | **0.01** | **0.01** | **0.01** | **0.01** | **0.03** | **0.01** |
| | **CQ** | **0.07** | **0.07** | **0.07** | **0.12** | **0.12** | **0.12** | **0.50** | **0.25** |
| **(g) organic-inorganic composite filler** | **O1** | **9.6** | **3.9** | **8.6** | | **5.4** | **5.5** | **5.0** | **2.5** |
| | **O2** | | | | | | | | |
| | **O3** | | | | | | | | |
| | **O4** | | | | | | | | |
| **Others** | **Fuselex X** | | | | | | | | |
| | **Aerosil R972** | | | | **1.9** | **2.8** | **1.6** | | |
| **Total (mass %)** | | **100.00** | **100.00** | **100.00** | **100.00** | **100.00** | **100.00** | **100.00** | **100.00** |
| **Total content \|(e)+(g)\| (mass%)** | | **13.7** | **13.3** | **11.7** | **9.0** | **9.0** | **8.6** | **9.0** | **4.5** |
| **Mass ratio \|(g)/(e)\|** | | **2.34** | **0.41** | **2.77** | **-** | **1.50** | **1.77** | **1.25** | **1.25** |
| **Mixability** | | **A** | **C** | **A** | **C** | **A** | **A** | **B** | **A** |
| **Stringiness of mixed material** | | **A** | **A** | **A** | **A** | **A** | **A** | **A** | **A** |
| **Shaping start time (sec)** | | **20** | **20** | **20** | **-** | - | **20** | - | - |
| **Evaluation** | | **A** | **A** | **A** | **-** | - | **A** | - | - |
| **Compressive strength (Mpa)** | | **200** | **209** | **201** | **115** | **109** | **192** | **87** | **114** |
| **Evaluation** | | **A** | **A** | **A** | **A** | **A** | **A** | **B** | **A** |
| **Contrast ratio** | | **0.69** | **0.64** | **0.70** | **0.64** | **0.65** | **0.65** | **0.68** | **0.68** |
| **Evaluation** | | **B** | **A** | **C** | **A** | **A** | **A** | **B** | **B** |

| | | **Example 39** | **Example 40** | **Example 41** | **Example 42** | **Example 43** | **Example 44** | **Example 45** |
|---|---|---|---|---|---|---|---|---|
| **Type** | | **Paste** | **Powder Liquid** | **Powder Liquid** | **Powder Liquid** | **Powder Liquid** | **Powder Liquid** | **Powder Liquid** |
| **Powder or First paste** | | **AP2** | **0** | **0** | **0** | **0** | **0** | **0** |
| **Liquid or Second paste** | | **BP2** | **L1** | **L1** | **L1** | **L1** | **L5** | **L5** |
| **Powder-Liquid ratio (mass ratio) or Paste ratio (mass ratio)** | | **1.4/1.0** | **3.6/1.0** | **2.0/1.0** | **2.0/1.0** | **3.6/1.0** | **4.7/1.0** | **2.0/1.0** |
| **Use** | | **Luting** | **Filling** | **Luting** | **Luting** | **Filling** | **Filling** | **Luting** |
| **(a) acid-reactive glass powder** | **G1** | | **7.8** | **6.7** | | | | |
| | **G2** | **29.2** | **58.1** | **49.4** | **44.6** | **55.8** | **69.4** | **56.2** |
| | **G3** | | | | | | | |
| | **G4** | | | | **6.7** | **7.8** | | |
| **(b) polyalkenoic acid** | **PCA1** | **10.4** | **3.6** | **5.5** | **5.5** | **3.6** | **0.2** | **0.3** |
| | **PCA2** | | | | | | | |
| | **PCA3** | | | | | | | |
| **(c)water** | **IEW** | **13.3** | **4.8** | **7.4** | **7.4** | **4.8** | **2.3** | **4.3** |
| **(d) polymeraizable monomer** | **GDMA** | **12.3** | **4.3** | **6.7** | **6.7** | **4.3** | **3.5** | **6.7** |
| | **HEMA** | **12.3** | **4.3** | **6.7** | **6.7** | **4.3** | **7.0** | **13.3** |
| | **4-AET** | **4.2** | **2.2** | **3.3** | **3.3** | **2.2** | **2.1** | **4.0** |
| | **FAM** | **4.2** | **2.4** | **3.7** | **3.7** | **2.4** | **2.3** | **4.3** |
| | **Bis-GMA** | **2.0** | | | | | | |
| **(e) porous inorganic filler** | **PIF1** | | **5.5** | **4.7** | **6.7** | **6.3** | **5.8** | **4.7** |
| | **PIF2** | **3.5** | | | | | | |
| | **PIF3** | | | | | | | |
| | **PIF4** | | | | | | | |
| | **PIF5** | | | | | | | |
| | **PIF6** | | | | | | | |
| | **PIF7** | | | | | | | |
| | **PIF8** | | | | | | | |
| **(f) polymerization initiator** | **KPS** | **0.01** | **0.02** | **0.01** | **0.05** | **0.05** | **0.02** | **0.01** |
| | **TSNa** | **0.45** | **0.60** | **0.48** | **0.50** | **0.53** | **0.59** | **0.55** |
| | **AA** | **0.01** | **0.01** | **0.01** | **0.05** | **0.05** | **0.01** | **0.01** |
| | **CQ** | **0.13** | **0.07** | **0.10** | **0.10** | **0.07** | **0.18** | **0.33** |
| **(g) organic-inorganic composite filler** | **O1** | **5.3** | **6.3** | **5.3** | **8.0** | **7.8** | **6.6** | **5.3** |
| | **O2** | | | | | | | |
| | **O3** | | | | | | | |
| | **O4** | | | | | | | |
| **Others** | **Fuselex X** | | | | | | | |
| | **Aerosil R972** | **2.7** | | | | | | |
| **Total (mass %)** | | **100.00** | **100.00** | **100.00** | **100.00** | **100.00** | **100.00** | **100.00** |
| **Total content \|(e) +(e)\| (mass%)** | | **8.8** | **11.8** | **10.0** | **14.7** | **14.1** | **12.4** | **10.0** |
| **Mass ratio \|(g)/(e)\|** | | **1.51** | **1.15** | **1.13** | **1.19** | **1.24** | **1.14** | **1.13** |
| **Mixability** | | **A** | **A** | **A** | **A** | **A** | **A** | **A** |
| **Stringiness of mixed material** | | **A** | **A** | **A** | **A** | **A** | **A** | **A** |
| **Shaping start time (sec)** | | **-** | **20** | **-** | **-** | **20** | **20** | **-** |
| **Evaluation** | | **-** | **A** | **-** | **-** | **A** | **A** | **-** |
| **Compressive strength (Mpa)** | | **86** | **211** | **121** | **111** | **196** | **177** | **91** |
| **Evaluation** | | **B** | **A** | **A** | **A** | **A** | **B** | **B** |
| **Contrast ratio** | | **0.65** | **0.64** | **0.65** | **0.65** | **0.64** | **0.65** | **0.65** |
| **Evaluation** | | **A** | **A** | **A** | **A** | **A** | **A** | **A** |

### Evaluation results of Examples 46 to 60

**[Table 11]**

| | | **Example 46** | **Example 47** | **Example 48** | **Example 49** | **Example 50** | **Example 51** | **Example 52** | **Example 53** |
|---|---|---|---|---|---|---|---|---|---|
| **Type** | | **Powder Liquid** | **Powder Liquid** | **Powder Liquid** | **Powder Liquid** | **Powder Liquid** | **Powder Liquid** | **Powder Liquid** | **Powder Liquid** |
| **Powder or First paste** | | **P32** | **P32** | **P8** | **P8** | **P8** | **P8** | **P8** | **P8** |
| **Liquid or Second paste** | | **L6** | **L6** | **L7** | **L7** | **L8** | **L8** | **L9** | **L10** |
| **Powder-Liquid ratio (mass ratio) or Paste ratio (mass ratio)** | | **2.5/1.0** | **2.0/1.0** | **3.6/1.0** | **2.0/1.0** | **3.6/1.0** | **2.0/1.0** | **1.2/1.0** | **3.6/1.0** |
| **Use** | | **Filling** | **Luting** | **Filling** | **Luting** | **Filling** | **Luting** | **Luting** | **Filling** |
| **(a) acid-reactive glass powder** | **G1** | | | | | | | | |
| | **G2** | **50.0** | **46.7** | **65.9** | **56.1** | **65.9** | **56.1** | **45.9** | **65.9** |
| | **G3** | | | | | | | | |
| | **G4** | | | | | | | | |
| **(b) polyalkenoic acid** | **PCA1** | **17.1** | **18.3** | | | **2.2** | **3.3** | **9.1** | **2.2** |
| | **PCA2** | | | **3.6** | **5.5** | **1.4** | **2.2** | | |
| | **PCA3** | | | | | | | | **1.4** |
| **(c) water** | **IEW** | **8.6** | **10.0** | **4.8** | **7.4** | **4.8** | **7.4** | **27.3** | **4.8** |
| **(d) polymeraizable monomer** | **GDMA** | **2.9** | **3.3** | **4.3** | **6.7** | **4.3** | **6.7** | **3.4** | **4.3** |
| | **HEMA** | **4.3** | **5.0** | **4.3** | **6.7** | **4.3** | **6.7** | **4.5** | **4.3** |
| | **4-AET** | **2.1** | **2.3** | **2.2** | **3.3** | **2.2** | **3.3** | **0.5** | **2.2** |
| | **FAM** | **1.3** | **1.5** | **2.4** | **3.7** | **2.4** | **3.7** | **0.5** | **2.4** |
| | **Bis-GMA** | | | | | | | | |
| **(e) porous inorganic filler** | **PIF1** | **5.7** | **5.3** | **5.5** | **4.7** | **5.5** | **4.7** | **3.8** | **5.5** |
| | **PIF2** | | | | | | | | |
| | **PIF3** | | | | | | | | |
| | **PIF4** | | | | | | | | |
| | **PIF5** | | | | | | | | |
| | **PIF6** | | | | | | | | |
| | **PIF7** | | | | | | | | |
| | **PIF8** | | | | | | | | |
| **(f) polymerization initiator** | **KPS** | **0.04** | **0.03** | **0.02** | **0.01** | **0.02** | **0.01** | **0.01** | **0.02** |
| | **TSNa** | **0.68** | **0.67** | **0.60** | **0.48** | **0.60** | **0.48** | **0.35** | **0.60** |
| | **AA** | **0.04** | **0.03** | **0.01** | **0.01** | **0.01** | **0.01** | **0.01** | **0.01** |
| | **CQ** | **0.14** | **0.17** | **0.07** | **0.10** | **0.07** | **0.10** | **0.23** | **0.07** |
| **(g) organic-inorganic composite filler** | **O1** | **7.1** | **6.7** | **6.3** | **5.3** | **6.3** | **5.3** | **4.4** | **6.3** |
| | **O2** | | | | | | | | |
| | **O3** | | | | | | | | |
| | **O4** | | | | | | | | |
| **Others** | **Fuselex X** | | | | | | | | |
| | **Aerosil R972** | | | | | | | | |
| **Total (mass %)** | | **100.00** | **100.00** | **100.00** | **100.00** | **100.00** | **100.00** | **100.00** | **100.00** |
| **Total content \|(e) + (e)\| (mass%)** | | **12.8** | **12.0** | **11.8** | **10.0** | **11.8** | **10.0** | **8.2** | **11.8** |
| **Mass ratio \|(g)/(e)\|** | | **1.25** | **1.26** | **1.15** | **1.13** | **1.15** | **1.13** | **1.16** | **1.15** |
| **Mixability** | | **B** | **B** | **A** | **A** | **A** | **A** | **A** | **B** |
| **Stringiness of mixed material** | | **A** | **A** | **A** | **A** | **A** | **A** | **A** | **A** |
| **Shaping start time (sec)** | | **20** | - | **20** | - | **20** | - | - | **20** |
| **Evaluation** | | **A** | - | **A** | - | **A** | - | - | **A** |
| **Compressive strength (Mpa)** | | **215** | **120** | **165** | **86** | **176** | **96** | **90** | **201** |
| **Evaluation** | | **A** | **A** | **B** | **B** | **B** | **B** | **B** | **A** |
| **Contrast ratio** | | **0.65** | **0.65** | **0.65** | **0.64** | **0.65** | **0.64** | **0.69** | **0.64** |
| **Evaluation** | | **A** | **A** | **A** | **A** | **A** | **A** | **B** | **A** |

| | | **Example 54** | **Example 55** | **Example 56** | **Example 57** | **Example 58** | **Example 59** | **Example 60** |
|---|---|---|---|---|---|---|---|---|
| **Type** | | **Powder Liquid** | **Powder Liquid** | **Powder Liquid** | **Powder Liquid** | **Powder Liquid** | **Powder Liquid** | **Powder Liquid** |
| **Powder or First paste** | | **P8** | **P8** | **P44** | **P45** | **P46** | **P47** | **P8** |
| **Liquid or Second paste** | | **L11** | **L12** | **L1** | **L1** | **L1** | **L1** | **L13** |
| **Powder-Liquid ratio (mass ratio) or Paste ratio (mass ratio)** | | **2.0/1.0** | **3.6/1.0** | **3.6/1.0** | **3.6/1.0** | **3.6/1.0** | **3.6/1.0** | **3.6/1.0** |
| **Use** | | **Lutine** | **Filling** | **Filling** | **Filling** | **Filling** | **Filling** | **Filling** |
| **(a) acid-reactive glass powder** | **G1** | | | | | | | |
| | **G2** | **56.1** | **65.8** | **69.9** | **69.9** | **69.9** | **69.9** | **65.8** |
| | **G3** | | | | | | | |
| | **G4** | | | | | | | |
| **(b) polyalkenoic acid** | **PCA1** | | **2.2** | **3.6** | **3.6** | **3.6** | **3.6** | **0.5** |
| | **PCA2** | | | | | | | |
| | **PCA3** | **5.5** | | | | | | |
| **(c) water** | **IEW** | **7.4** | **4.8** | **4.8** | **4.8** | **4.8** | **4.8** | **2.2** |
| **(d) polymeraizable monomer** | **GDMA** | **6.7** | **5.0** | **4.3** | **4.3** | **4.3** | **4.3** | **7.0** |
| | **HEMA** | **6.7** | **5.0** | **4.3** | **4.3** | **4.3** | **4.3** | **7.0** |
| | **4-AET** | **3.3** | **2.3** | **2.2** | **2.2** | **2.2** | **2.2** | **2.3** |
| | **FAM** | **3.7** | **2.4** | **2.4** | **2.4** | **2.4** | **2.4** | **2.7** |
| | **Bis-GMA** | | | | | | | |
| **(e) porous inorganic filler** | **PIF1** | **4.7** | **5.5** | | | | | **5.5** |
| | **PIF2** | | | | | | | |
| | **PIF3** | | | | | | | |
| | **PIF4** | | | | | | | |
| | **PIF5** | | | **7.8** | | | | |
| | **PIF6** | | | | **7.8** | | | |
| | **PIF7** | | | | | **7.8** | | |
| | **PIF8** | | | | | | **7.8** | |
| **(f) polymerization initiator** | **KPS** | **0.01** | **0.02** | **0.02** | **0.02** | **0.02** | **0.02** | **0.02** |
| | **TSNa** | **0.48** | **0.60** | **0.60** | **0.60** | **0.60** | **0.60** | **0.60** |
| | **AA** | **0.01** | **0.01** | **0.01** | **0.01** | **0.01** | **0.01** | **0.01** |
| | **CQ** | **0.10** | **0.07** | **0.07** | **0.07** | **0.07** | **0.07** | **0.07** |
| **(g) organic-inorganic composite filler** | **O1** | **5.3** | **6.3** | | | | | **6.3** |
| | **O2** | | | | | | | |
| | **O3** | | | | | | | |
| | **O4** | | | | | | | |
| **Others** | **Fuselex X** | | | | | | | |
| | **Aerosil R972** | | | | | | | |
| **Total (mass %)** | | **100.00** | **100.00** | **100.00** | **100.00** | **100.00** | **100.00** | **100.00** |
| **Total content \|(e) +(g)\| (mass%)** | | **10.0** | **11.8** | **7.8** | **7.8** | **7.8** | **7.8** | **11.8** |
| **Mass ratio \|(g)/(e)\|** | | **1.13** | **1.15** | **-** | **-** | **-** | | **1.15** |
| **Mixability** | | **B** | **A** | **C** | **C** | **C** | **C** | **A** |
| **Stringiness of mixed material** | | **A** | **A** | **A** | **A** | **A** | **A** | **A** |
| **Shaping start time (sec)** | | - | **20** | **20** | **20** | **20** | **20** | **20** |
| **Evaluation** | | - | **A** | **A** | **A** | **A** | **A** | **A** |
| **Compressive strength (Mpa)** | | **122** | **188** | **210** | **208** | **211** | **211** | **180** |
| **Evaluation** | | **A** | **A** | **A** | **A** | **A** | **A** | **A** |
| **Contrast ratio** | | **0.65** | **0.65** | **0.64** | **0.65** | **0.67** | **0.69** | **0.65** |
| **Evaluation** | | **A** | **A** | **A** | **A** | **A** | **B** | **A** |

### Evaluation results of Examples 61 to 63 and Comparative Examples 1 to 9

**[Table 12]**

| | | **Example 61** | **Example 62** | **Example 63** | **Comparative Example 1** | **Comparative Example 2** | **Comparative Example 3** | **Comparative Example 4** | **Comparative Example 5** |
|---|---|---|---|---|---|---|---|---|---|
| **Type** | | **Powder Liquid** | **Powder Liquid** | **Powder Liquid** | **Powder Liquid** | **Paste** | **Powder Liquid** | **Powder Liquid** | **Powder Liquid** |
| **Powder or First paste** | | **P8** | **P48** | **P9** | **P37** | **AP3** | **P38** | **P39** | **P40** |
| **Liquid or Second paste** | | **L14** | **L1** | **L1** | **L1** | **BP1** | **L1** | **L1** | **L1** |
| **Powder-Liquid ratio (mass ratio) or Paste ratio (mass ratio)** | | **3.6/1.0** | **3.6/1.0** | **3.6/1.0** | **3.6/1.0** | **1.5/1.0** | **1.0/1.0** | **3.6/1.0** | **3.6/1.0** |
| **Use** | | **Filling** | **Filling** | **Filling** | **Filling** | **Lutine** | **Lutine** | **Filling** | **Filling** |
| **(a) acid-reactive glass powder** | **G1** | | | | | | | | |
| | **G2** | **65.7** | **75.7** | **65.9** | **77.7** | **42.6** | **49.1** | **62.0** | **66.0** |
| | **G3** | | | | | | | | **11.7** |
| | **G4** | | | | | | | | |
| **(b) polyalkenoic acid** | **PCA1** | **3.0** | **3.6** | **3.6** | **3.6** | **6.0** | **8.3** | **3.6** | **3.6** |
| | **PCA2** | | | | | | | | |
| | **PCA3** | | | | | | | | |
| **(c) water** | **IEW** | **4.4** | **4.8** | **4.8** | **4.8** | **8.8** | **11.1** | **4.8** | **4.8** |
| **(d) polymeraizable monomer** | **GDMA** | **4.9** | **4.3** | **4.3** | **4.3** | **14.9** | **10.0** | **4.3** | **4.3** |
| | **HEMA** | **4.9** | **4.3** | **4.3** | **4.3** | **14.9** | **10.0** | **4.3** | **4.3** |
| | **4-AET** | **2.2** | **2.2** | **2.2** | **2.2** | **3.8** | **5.0** | **2.2** | **2.2** |
| | **FAM** | **2.4** | **2.4** | **2.4** | **2.4** | **4.2** | **5.5** | **2.4** | **2.4** |
| | **Bis-GMA** | | | | | **1.4** | | | |
| **(e) porous inorganic filler** | **PIF1** | **5.5** | **1.1** | **5.5** | | | **0.5** | **15.7** | |
| | **PIF2** | | | | | | | | |
| | **PIF3** | | | | | | | | |
| | **PIF4** | | | | | | | | |
| | **PIF5** | | | | | | | | |
| | **PIF6** | | | | | | | | |
| | **PIF7** | | | | | | | | |
| | **PIF8** | | | | | | | | |
| **(f) polymerization initiator** | **KPS** | **0.02** | **0.02** | **0.02** | **0.02** | **0.01** | **0.01** | **0.02** | **0.02** |
| | **TSNa** | **0.60** | **0.60** | **0.60** | **0.60** | **0.46** | **0.33** | **0.60** | **0.60** |
| | **AA** | **0.01** | **0.01** | **0.01** | **0.01** | **0.01** | **0.01** | **0.01** | **0.01** |
| | **CQ** | **0.07** | **0.07** | **0.07** | **0.07** | **0.12** | **0.15** | **0.07** | **0.07** |
| **(g) organic-inorganic composite filler** | **O1** | **6.3** | **0.9** | | | | | | |
| | **O2** | | | **6.3** | | | | | |
| | **O3** | | | | | | | | |
| | **O4** | | | | | | | | |
| **Others** | **Fuselex X** | | | | | | | | |
| | **Aerosil R972** | | | | | **2.8** | | | |
| **Total (mass %)** | | **100.00** | **100.00** | **100.00** | **100.00** | **100.00** | **100.00** | **100.00** | **100.00** |
| **Total content \|(e) + (g)\| (mass%)** | | **11.8** | **2.0** | **11.8** | **-** | **-** | **0.5** | **15.7** | **-** |
| **Mass ratio \|(g)/(e)\|** | | **1.15** | **0.82** | **1.15** | **-** | **-** | **-** | **-** | **-** |
| **Mixability** | | **A** | **A** | **A** | **A** | **A** | **A** | **D** | **A** |
| **Stringiness of mixed material** | | **A** | **B** | **A** | **C** | **C** | **C** | **A** | **B** |
| **Shaping start time (sec)** | | **20** | **30** | **20** | **50** | **-** | **-** | **20** | **30** |
| **Evaluation** | | **A** | **B** | **A** | **D** | **-** | **-** | **A** | **B** |
| **Compressive strength (Mpa)** | | **212** | **219** | **213** | **223** | **196** | **130** | **168** | **149** |
| **Evaluation** | | **A** | **A** | **A** | **A** | **A** | **A** | **B** | **D** |
| **Contrast ratio** | | **0.65** | **0.64** | **0.65** | **0.64** | **0.63** | **0.64** | **0.65** | **0.63** |
| **Evaluation** | | **A** | **A** | **A** | **A** | **A** | **A** | **A** | **A** |

| | | **Comparative Example 6** | **Comparative Example 7** | **Comparative Example 8** | **Comparative Example 9** |
|---|---|---|---|---|---|
| **Type** | | **Powder Liquid** | **Paste** | **Powder Liquid** | **Powder Liquid** |
| **Powder or First paste** | | **P41** | **AP5** | **P42** | **P43** |
| **Liquid or Second paste** | | **L1** | **BP1** | **L1** | **L1** |
| **Powder-Liquid ratio (mass ratio) or Paste ratio (mass ratio)** | | **3.8/1.0** | **1.5/1.0** | **1.0/1.0** | **3.6/1.0** |
| **Use** | | **Filling** | **Luting** | **Luting** | **Filling** |
| **(a) acid-re active glass powder** | **G1** | | | | |
| | **G2** | **66.6** | **33.6** | **44.6** | **68.3** |
| | **G3** | | | | |
| | G4 | | | | |
| **(b) polyalkenoic acid** | **PCA1** | **3.4** | **6.0** | **8.3** | **3.6** |
| | **PCA2** | | | | |
| | **PCA3** | | | | |
| **(c) water** | **IEW** | **4.6** | **8.8** | **11.1** | **4.8** |
| **(d) polymeraizable monomer** | **GDMA** | **4.2** | **14.9** | **10.0** | **4.3** |
| | **HEMA** | **4.2** | **14.9** | **10.0** | **4.3** |
| | **4-AET** | **2.1** | **3.8** | **5.0** | **2.2** |
| | **FAM** | **2.3** | **4.2** | **5.5** | **2.4** |
| | **Bis-GMA** | | **1.4** | | |
| **(e) porous inorganic filler** | **PIF1** | | | | |
| | **PIF2** | | | | |
| | **PIF3** | | | | |
| | **PIF4** | | | | |
| | **PIF5** | | | | |
| | **PIF6** | | | | |
| | **PIF7** | | | | |
| | **PIF8** | | | | |
| **(f) polymerization initiator** | **KPS** | **0.02** | **0.01** | **0.01** | **0.02** |
| | **TSNa** | **0.61** | **0.46** | **0.33** | **0.60** |
| | **AA** | **0.01** | **0.01** | **0.01** | **0.01** |
| | **CQ** | **0.06** | **0.12** | **0.15** | **0.07** |
| **(g) organic-inorganic composite filler** | **O1** | | **9.0** | **5.0** | **9.4** |
| | **O2** | | | | |
| | **O3** | | | | |
| | **O4** | | | | |
| **Others** | **Fuselex X** | **11.9** | | | |
| | **Aerosil R972** | | **2.8** | | |
| **Total (mass %)** | | **100.00** | **100.00** | **100.00** | **100.00** |
| **Total content \|(e) + (g)\| (mass%)** | | - | **9.0** | **5.0** | **9.4** |
| **Mass ratio \|(g)/(e)\|** | | - | - | **-** | **-** |
| **Mixability** | | **A** | **A** | **A** | **A** |
| **Stringiness of mixed material** | | **C** | **A** | **A** | **A** |
| **Shaping start time (see)** | | **50** | - | **-** | **20** |
| **Evaluation** | | **D** | - | **-** | **A** |
| **Compressive strength (Mpa)** | | **148** | **113** | **118** | **218** |
| **Evaluation** | | **D** | **A** | **A** | **A** |
| **Contrast ratio** | | **0.63** | **0.72** | **0.71** | **0.73** |
| **Evaluation** | | **A** | **D** | **D** | **D** |

### <Evaluation results of Examples: for filling>

In Examples 1 to 5, 8 to 10, 12 to 33, 36, 40, 43, 44, 46, 48, 50, 53 and 55 to 63, there was little stringiness immediately after mixing, and it was possible to perform shaping operations soon after filling the simulated cavity. Furthermore, good mixability, mechanical characteristic and transparency exhibited, and thus these Examples had desirable properties as a dental resin-reinforced glass ionomer cement composition for filling. Among these, when the (e) porous inorganic filler and the (g) organic-inorganic composite filler were both contained within the preferable range or a more preferable range, more preferable mixability was exhibited.

### <Evaluation results of Examples: for luting>

In Examples 6, 7, 11, 34, 35, 37 to 39, 41, 42, 45, 47, 49, 51, 52 and 54, there was little stringiness immediately after mixing. Furthermore, good mixability, mechanical characteristic and transparency exhibited, and thus these Examples had desirable properties as a dental resin-reinforced glass ionomer cement composition for luting. Among these, when the (e) porous inorganic filler and the (g) organic-inorganic composite filler were both contained within the preferable range or a more preferable range, more preferable mixability was exhibited.

### <Evaluation results of Comparative Examples>

### <Comparative Example 1>

The dental resin-reinforced glass ionomer cement composition for filling of Comparative Example 1 did not contain the (e) porous inorganic filler. As a result of evaluating Comparative Example 1, significant stringiness was observed in immediately after mixing and a long time was required until shaping operation became possible.

### <Comparative Example 2>

The dental resin-reinforced glass ionomer cement composition for luting of Comparative Example 2 did not contain the (e) porous inorganic filler. As a result of evaluating Comparative Example 2, significant stringiness was observed in immediately after mixing.

### <Comparative Example 3>

The dental resin-reinforced glass ionomer cement composition for luting of Comparative Example 3 had a small content of the (e) porous inorganic filler. As a result of evaluating Comparative Example 3, significant stringiness was observed in immediately after mixing.

### <Comparative Example 4>

The dental resin-reinforced glass ionomer cement composition for filling of Comparative Example 4 had a large content of the (e) porous inorganic filler. As a result of evaluating Comparative Example 3, mixability was poor.

### <Comparative Example 5>

The dental resin-reinforced glass ionomer cement composition for filling of Comparative Example 5 did not contain the (e) porous inorganic filler, but instead contained the (a) acid-reactive glass powder (G3) having a large particle diameter. As a result of evaluating Comparative Example 5, the compressive strength was low.

### <Comparative Example 6>

The dental resin-reinforced glass ionomer cement composition for filling of Comparative Example 6 did not contain the (e) porous inorganic filler, but instead contained the non acid-reactive inorganic powder having no pores. As a result of evaluating Comparative Example 6, significant stringiness was observed immediately after mixing, a long time was required until shaping operation became possible, and the compressive strength was low.

### <Comparative Examples 7 and 8>

The dental resin-reinforced glass ionomer cement composition for luting of Comparative Examples 7 and 8 did not contain the (e) porous inorganic filler, and contained only the (g) organic-inorganic composite filler. As a result of evaluating Comparative Examples 7 and 8, the contrast ratio was high and it was opaque.

### <Comparative Example 9>

The dental resin-reinforced glass ionomer cement composition for filling of Comparative Example 9 did not contain the (e) porous inorganic filler, and contained only the (g) organic-inorganic composite filler. As a result of evaluating Comparative Example 9, the contrast ratio was high and it was opaque.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### Industrial applicability

The dental resin-reinforced glass ionomer cement composition of the present invention can be suitably used in dental treatments such as filling dental cavities, lining layers or linings, luting dental prosthetic devices such as crown, inlay and bridge to tooth substance, pit and fissure sealant, preventive coating of tooth surfaces, and abutment construction.

## Claims

1. A dental resin-reinforced glass ionomer cement composition comprising
(a) acid-reactive glass powder,
(b) polyalkenoic acid,
(c) water,
(d) polymeraizable monomer,
(e) porous inorganic filler: 1 % by mass or more and 15 % by mass or less, and
(f) polymerization initiator, wherein
the center portion of the (e) porous inorganic filler is an inorganic particle consisting of only silicon dioxide or consisting of silicon dioxide and an oxide containing one or more metal elements.

2. The dental resin-reinforced glass ionomer cement composition according to claim 1, wherein
the (e) porous inorganic filler has a 50 % particle diameter (D50) within a range of 0.1 µm or more and 10 µm or less, a pore volume within a range of 0.01 cc/g or more and 1.00 cc/g or less, and a specific surface area within a range of 5 m²/g or more and 500 m²/g or less.

3. The dental resin-reinforced glass ionomer cement composition according to claim 1 or 2, wherein
the dental resin-reinforced glass ionomer cement composition further comprises (g) organic-inorganic composite filler,
the (g) organic-inorganic composite filler has a 50 % particle diameter (D50) within a range of 1 µm or more, and
the (g) organic-inorganic composite filler contains 10 mass % or more and 85 mass % or less of an inorganic filler having a 50% particle diameter (D50) of 0.01 µm or more and 3 µm or less.

4. The dental resin-reinforced glass ionomer cement composition according to claim 3, wherein
a total content of the (e) porous inorganic filler and the (g) organic-inorganic composite filler is 2 % by mass or more and 20 % by mass or less based on the whole of the dental resin-reinforced glass ionomer cement composition, and
the mass ratio of the content of the (e) porous inorganic filler to the content of the (g) organic-inorganic composite filler [(g) organic-inorganic composite filler/(e) porous inorganic filler] is 0.5 or more and 2.5 or less.

5. The dental resin-reinforced glass ionomer cement composition according to claim 1 or 2, wherein
the dental resin-reinforced glass ionomer cement composition comprises
(a) an acid-reactive glass powder: 30 % by mass or more and 80 % by mass or less,
(b) a polyalkenoic acid: 0.5 % by mass or more and 17 % by mass or less,
(c) water: 0.5 % by mass or more and 27 % by mass or less, and
(d) polymeraizable monomer: 2 % by mass or more and 48 % by mass or less.

6. The dental resin-reinforced glass ionomer cement composition according to claim 3, wherein
the dental resin-reinforced glass ionomer cement composition comprises
(a) an acid-reactive glass powder: 30 % by mass or more and 80 % by mass or less,
(b) a polyalkenoic acid: 0.5 % by mass or more and 17 % by mass or less,
(c) water: 0.5 % by mass or more and 27 % by mass or less, and
(d) polymeraizable monomer: 2 % by mass or more and 48 % by mass or less.

7. The dental resin-reinforced glass ionomer cement composition according to claim 4, wherein
the dental resin-reinforced glass ionomer cement composition comprises
(a) an acid-reactive glass powder: 30 % by mass or more and 80 % by mass or less,
(b) a polyalkenoic acid: 0.5 % by mass or more and 17 % by mass or less,
(c) water: 0.5 % by mass or more and 27 % by mass or less, and
(d) polymeraizable monomer: 2 % by mass or more and 48 % by mass or less.

8. The dental resin-reinforced glass ionomer cement composition according to claim 1 or 2, wherein
shaping start time of the dental resin-reinforced glass ionomer cement composition is within 40 seconds.

9. The dental resin-reinforced glass ionomer cement composition according to claim 3, wherein
shaping start time of the dental resin-reinforced glass ionomer cement composition is within 40 seconds.

10. The dental resin-reinforced glass ionomer cement composition according to claim 4, wherein
shaping start time of the dental resin-reinforced glass ionomer cement composition is within 40 seconds.

11. The dental resin-reinforced glass ionomer cement composition according to claim 5, wherein
shaping start time of the dental resin-reinforced glass ionomer cement composition is within 40 seconds.

12. The dental resin-reinforced glass ionomer cement composition according to claim 6, wherein
shaping start time of the dental resin-reinforced glass ionomer cement composition is within 40 seconds.

13. The dental resin-reinforced glass ionomer cement composition according to claim 7, wherein
shaping start time of the dental resin-reinforced glass ionomer cement composition is within 40 seconds.
